(19) Europäisches Patentamt
European Patent Office
Office européen des brevets

(11) EP 4 134 081 A1

(12) EUROPEAN PATENT APPLICATION
published in accordance with Art. 153(4) EPC

(43) Date of publication:
**15.02.2023 Bulletin 2023/07**

(21) Application number: **21784104.8**

(22) Date of filing: **09.04.2021**

(51) International Patent Classification (IPC):
***A61K 31/519*** *(2000.01)* ***A61K 31/4184*** *(2000.01)*
***A61K 31/4523*** *(2000.01)* ***A61K 45/00*** *(1974.07)*
***A61P 35/00*** *(2000.01)* ***A61P 43/00*** *(2000.01)*
***C07D 487/04*** *(1974.07)*

(52) Cooperative Patent Classification (CPC):
**A61K 31/4184; A61K 31/4523; A61K 31/519; A61K 45/00; A61P 35/00; A61P 43/00; C07D 487/04**

(86) International application number:
**PCT/JP2021/015032**

(87) International publication number:
**WO 2021/206167 (14.10.2021 Gazette 2021/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.04.2020 JP 2020071355**

(71) Applicant: **Taiho Pharmaceutical Co., Ltd.**
**Chiyoda-ku, Tokyo 101-8444 (JP)**

(72) Inventors:
- **HIRAI, Hiroshi**
  **Tokyo 101-8444 (JP)**
- **MATSUOKA, Kazuaki**
  **Tokyo 101-8444 (JP)**
- **MIURA, Akihiro**
  **Tokyo 101-8444 (JP)**

(74) Representative: **Hoffmann Eitle**
**Patent- und Rechtsanwälte PartmbB**
**Arabellastraße 30**
**81925 München (DE)**

# (54) CANCER THERAPY USING 3,5-DISUBSTITUTED BENZENE ALKYNYL COMPOUND AND MEK INHIBITOR

(57) Provided is a novel combination therapy that exhibits excellent antitumor effects, and that uses an FGFR-inhibiting compound and an MEK inhibitor. An antitumor agent contains (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as an active ingredient and is administered in combination with an MEK inhibitor.

Fig. 2

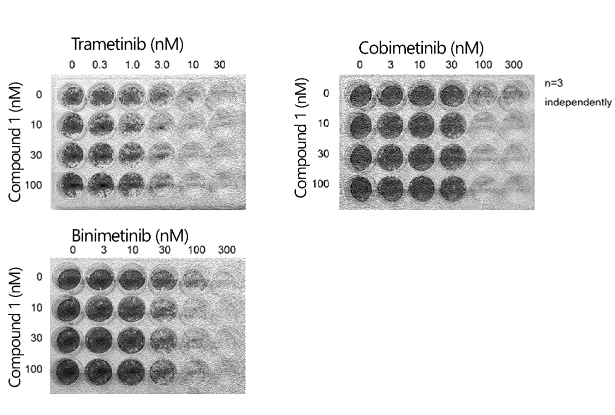


EP 4 134 081 A1

Processed by Luminess, 75001 PARIS (FR)

**Description**

Technical Field

Cross-Reference to Related Patent Applications

This application claims priority based on Japanese Patent Application No. 2020-071355 filed on April 10, 2020, the entire content of which is incorporated herein by reference. The present disclosure relates to an antitumor agent, an antitumor effect enhancer, and a kit preparation.

Background Art

Fibroblast growth factors (FGFs) are expressed in various tissues and are one of the growth factors that regulate cell proliferation and differentiation. The physiological activity of FGFs is mediated by fibroblast growth factor receptors (FGFRs), which are specific cell surface receptors. FGFRs belong to a receptor protein tyrosine kinase family and contain an extracellular ligand-binding domain, a single transmembrane domain, and an intracellular tyrosine kinase domain. Four types of FGFRs (FGFR1, FGFR2, FGFR3, and FGFR4) have been identified. FGFRs bind to FGFs to form dimers and are activated by phosphorylation. Activation of the receptors induces mobilization and activation of specific downstream signal transduction molecules, thereby developing physiological functions. Some studies report the relationship between aberrant FGF/FGFR signaling and various human tumors. Aberrant activation of FGF/FGFR signaling in human tumors is considered to be due to overexpression of FGFRs and/or gene amplification, gene mutation, chromosomal translocation, insertion, inversion, gene fusion, or an autocrine or paracrine mechanism by overproduction of FGFs (ligands) (NPL 1, 2, 3, and 4). (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one, which is a 3,5-disubstituted benzene alkynyl compound (in the present specification, "compound 1"), or a salt thereof is known as an FGFR inhibitor. Combinations of FGFR inhibitors and other antitumor agents have been reported (PTL 2 and PTL 3).

RAS genes are a factor playing a central role in the RAS/MAPK kinase pathway that promotes cell proliferation. RAS genes become inactive when GDP binds, and they become active when GDP is exchanged by GTP by means of guanine nucleotide exchange factors (GEFs). This induces the mobilization and activation of specific signal transduction molecules downstream and allows for the development of physiological functions. Additionally, when activated RAS genes bind to GTPase-activating proteins (GAP) to hydrolyze GTP to GDP, they become inactive.

RAS genes are known to be oncogenes, and active mutations of RAS genes have been reported in various types of cancer, such as bowel cancer, lung cancer, and pancreatic cancer. Forcibly expressing RAS genes with these active mutations in normal cells transforms the cells. It is known that cancers with active mutations of RAS genes are resistant to molecularly targeted therapies, and inhibitors of receptor kinases or RAS/MAPK kinase pathway components (e.g., EGFR inhibitors, MEK inhibitors, ERK inhibitors) have limited clinical efficacy on cancers with active mutations of RAS genes. For example, the effects of EGFR inhibitors on KRAS-mutated bowel cancer or the effects of MEK inhibitors on KRAS-mutated lung cancer are limited.

Recent research has revealed that treatment of a KRAS-mutated lung cancer cell line with an MEK inhibitor activates a growth signaling pathway mediated by FGF receptors. From this result, it is hoped that administration of an FGFR inhibitor and an MEK inhibitor in combination will provide antitumor effects. There are reports on the administration of an FGFR inhibitor and an MEK inhibitor in combination, such as using an MEK inhibitor in combination with an RTK inhibitor (PTL 3) and a combination of an MEK inhibitor with a therapeutic agent for KRAS-mutated cancer, which targets only mesenchymal tumors (PTL 4). In such circumstances, therapeutic methods for cancer other than those using these combinations are still eagerly awaited.

Citation List

Patent Literature

PTL 1: WO2013/108809A
PTL 2: WO2017/150725A
PTL 3: WO2016/130917A
PTL 4: WO2017/086332A
PTL 5: WO2015/008839A

Non-patent Literature

NPL 1: Nat. Rev. Cancer 10:116-129 (2010)
NPL 2: J. Clin. Oncol. 24, 3664-3671 (2006)
NPL 3: Mol. Cancer Res. 3, 655-667 (2005)
NPL 4: Cancer Res. 70, 2085-2094 (2010)

Summary of Invention

Technical Problem

An object of the present disclosure is to provide a novel combination therapy that exhibits excellent antitumor effects and that uses an FGFR-inhibiting compound and an MEK inhibitor.

Solution to Problem

In view of such circumstances, the present inventors conducted research on the combination of a wide variety of compounds with FGFR-inhibiting activity and MEK inhibitors and found that the object can be achieved by a combination of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one, which is an FGFR inhibitor, or a salt thereof with an MEK inhibitor. Accordingly, the present disclosure provides the following Items [1] to [44] .

[1] An antitumor agent comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as an active ingredient, the antitumor agent being administered in combination with an MEK inhibitor.
[2] The antitumor agent according to [1], wherein the MEK inhibitor is trametinib or a salt thereof.
[3] The antitumor agent according to [1], wherein the MEK inhibitor is binimetinib or a salt thereof.
[4] The antitumor agent according to [1], wherein the MEK inhibitor is cobimetinib or a salt thereof.
[5] The antitumor agent according to [2], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered at a dose selected from the group consisting of 8 mg, 12 mg, 16 mg, and 20 mg per dose once daily, and trametinib or a salt thereof is administered at a dose selected from the group consisting of 1 mg, 1.5 mg, and 2 mg per dose once daily.
[6] The antitumor agent according to [3], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered at a dose selected from the group consisting of 8 mg, 12 mg, 16 mg, and 20 mg per dose once daily, and binimetinib or a salt thereof is administered at a dose selected from the group consisting of 30 mg and 45 mg per dose twice daily.
[7] The antitumor agent according to [4], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered at a dose selected from the group consisting of 8 mg, 12 mg, 16 mg, and 20 mg per dose once daily, and cobimetinib or a salt thereof is administered at a dose selected from the group consisting of 40 mg and 60 mg per dose once daily.
[8] The antitumor agent according to [2], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is intermittently administered at a dose selected from the group consisting of 24 mg, 36 mg, 56 mg, 80 mg, 120 mg, and 160 mg per dose, and trametinib or a salt thereof is administered at a dose selected from the group consisting of 1 mg, 1.5 mg, and 2 mg per dose once daily.
[9] The antitumor agent according to [3], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is intermittently administered at a dose selected from the group consisting of 24 mg, 36 mg, 56 mg, 80 mg, 120 mg, and 160 mg per dose, and binimetinib or a salt thereof is administered at a dose selected from the group consisting of 30 mg and 45 mg per dose twice daily.
[10] The antitumor agent according to [4], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is intermittently administered at a dose selected from the group consisting of 24 mg, 36 mg, 56 mg, 80 mg, 120 mg, and 160 mg per dose, and cobimetinib or a salt thereof is administered at a dose selected from the group consisting of 40 mg and 60 mg per dose once daily.
[11] The antitumor agent according to any one of [1] to [10], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered before, simultaneously with, or after administration of the MEK inhibitor.

[12] The antitumor agent according to any one of [1] to [11], wherein the tumor has an aberration in an RAS/MAPK signaling pathway.

[13] The antitumor agent according to any one of [1] to [12], wherein the tumor is at least one member selected from the group consisting of head and neck cancer, gastroenterological cancer, lung cancer, breast cancer, genital cancer, a hematopoietic organ tumor, a bone and soft tissue tumor, skin cancer, and a brain tumor.

[14] The antitumor agent according to any one of [1] to [13], wherein the tumor is lung cancer, pancreatic cancer, colorectal cancer, or a brain tumor.

[15] An antitumor effect enhancer for enhancing an antitumor effect of an MEK inhibitor, the antitumor effect enhancer comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as an active ingredient.

[16] An antitumor agent for use in the treatment of a cancer patient to whom an MEK inhibitor has been administered, the antitumor agent comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as an active ingredient.

[17] A pharmaceutical composition for use in the treatment of a tumor by administering the pharmaceutical composition in combination with an MEK inhibitor, the pharmaceutical composition comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and a pharmaceutical carrier as active ingredients.

[18] (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, for use in combination therapy with an MEK inhibitor in the treatment of a tumor.

[19] A combination of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and an MEK inhibitor for use in the treatment of a tumor.

[20] A method for treating a tumor, comprising administering a therapeutically effective amount of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and a therapeutically effective amount of an MEK inhibitor to a patient in need thereof.

[21] A method for enhancing an antitumor effect of an MEK inhibitor, comprising administering a therapeutically effective amount of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof to a patient to whom the MEK inhibitor has been administered.

[22] A method for treating a tumor, comprising administering a therapeutically effective amount of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof to a patient to whom an MEK inhibitor has been administered.

[23] Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in the treatment of a tumor in a combination therapy with an MEK inhibitor.

[24] Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in the enhancement of an antitumor effect of an MEK inhibitor.

[25] Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in the treatment of a cancer patient to whom an MEK inhibitor has been administered.

[26] Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in the production of an antitumor agent for use in a combination therapy with an MEK inhibitor.

[27] Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in the production of an antitumor effect enhancer for an MEK inhibitor.

[28] Use of a combination of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and an MEK inhibitor in the treatment of a tumor.

[29] Use of a combination of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and an MEK inhibitor in the production of an antitumor agent.

[30] A pharmaceutical composition comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and an MEK inhibitor as active ingredients.

[31] The pharmaceutical composition according to [30], for use in the treatment of a tumor.

[32] The antitumor effect enhancer according to [15], the antitumor agent according to [16], the pharmaceutical composition according to any one of [17], [30], and [31], the compound 1 or a salt thereof according to [18], the combination according to [19], the method according to any one of [20] to [22], or the use according to any one of [23] to [29], wherein the MEK inhibitor is trametinib or a salt thereof.

[33] The antitumor effect enhancer according to [15], the antitumor agent according to [16], the pharmaceutical composition according to any one of [17], [30], and [31], the compound 1 or a salt thereof according to [18], the combination according to [19], the method according to any one of [20] to [22], or the use according to any one of [23] to [29], wherein the MEK inhibitor is binimetinib or a salt thereof.

[34] The antitumor effect enhancer according to [15], the antitumor agent according to [16], the pharmaceutical

composition according to any one of [17], [30], and [31], the compound 1 or a salt thereof according to [18], the combination according to [19], the method according to any one of [20] to [22], or the use according to any one of [23] to [29], wherein the MEK inhibitor is cobimetinib or a salt thereof.

[35] The antitumor effect enhancer, the antitumor agent, the pharmaceutical composition, the compound 1 or a salt thereof, the combination, the method, or the use according to [32], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered at a dose selected from the group consisting of 8 mg, 12 mg, 16 mg, and 20 mg per dose once daily, and trametinib or a salt thereof is administered at a dose selected from the group consisting of 1 mg, 1.5 mg, and 2 mg per dose once daily.

[36] The antitumor effect enhancer, the antitumor agent, the pharmaceutical composition, the compound 1 or a salt thereof, the combination, the method, or the use according to [33], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered at a dose selected from the group consisting of 8 mg, 12 mg, 16 mg, and 20 mg per dose once daily, and binimetinib or a salt thereof is administered at a dose selected from the group consisting of 30 mg and 45 mg per dose twice daily.

[37] The antitumor effect enhancer, the antitumor agent, the pharmaceutical composition, the compound 1 or a salt thereof, the combination, the method, or the use according to [34], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered at a dose selected from the group consisting of 8 mg, 12 mg, 16 mg, and 20 mg per dose once daily, and cobimetinib or a salt thereof is administered at a dose selected from the group consisting of 40 mg and 60 mg per dose once daily.

[38] The antitumor effect enhancer, the antitumor agent, the pharmaceutical composition, the compound 1 or a salt thereof, the combination, the method, or the use according to [32], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is intermittently administered at a dose selected from the group consisting of 24 mg, 36 mg, 56 mg, 80 mg, 120 mg, and 160 mg per dose, and trametinib or a salt thereof is administered at a dose selected from the group consisting of 1 mg, 1.5 mg, and 2 mg per dose once daily.

[39] The antitumor effect enhancer, the antitumor agent, the pharmaceutical composition, the compound 1 or a salt thereof, the combination, the method, or the use according to [33], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is intermittently administered at a dose selected from the group consisting of 24 mg, 36 mg, 56 mg, 80 mg, 120 mg, and 160 mg per dose, and binimetinib or a salt thereof is administered at a dose selected from the group consisting of 30 mg and 45 mg per dose twice daily.

[40] The antitumor effect enhancer, the antitumor agent, the pharmaceutical composition, the compound 1 or a salt thereof, the combination, the method, or the use according to [34], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is intermittently administered at a dose selected from the group consisting of 24 mg, 36 mg, 56 mg, 80 mg, 120 mg, and 160 mg per dose, and cobimetinib or a salt thereof is administered at a dose selected from the group consisting of 40 mg and 60 mg per dose once daily.

[41] The antitumor effect enhancer according to [15], the antitumor agent according to [16], the pharmaceutical composition according to any one of [17], [30], and [31], the compound 1 or a salt thereof according to [18], the combination according to [19], the method according to any one of [20] to [22], the use according to any one of [23] to [29], or the antitumor effect enhancer, the antitumor agent, the pharmaceutical composition, the compound 1 or a salt thereof, the combination, the method, or the use according to any one of [32] to [40], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered before, simultaneously with, or after administration of the MEK inhibitor.

[42] The antitumor effect enhancer according to [15], the antitumor agent according to [16], the pharmaceutical composition according to any one of [17], [30], and [31], the compound 1 or a salt thereof according to [18], the combination according to [19], the method according to any one of [20] to [22], the use according to any one of [23] to [29], or the antitumor effect enhancer, the antitumor agent, the pharmaceutical composition, the compound 1 or a salt thereof, the combination, the method, or the use according to any one of [32] to [41], wherein the tumor has an aberration in a RAS/MAPK signaling pathway.

[43] The antitumor effect enhancer, the antitumor agent, the pharmaceutical composition, the compound 1 or a salt thereof, the combination, the method, or the use according to any one of [15] to [42], wherein the tumor is at least one member selected from the group consisting of head and neck cancer, gastroenterological cancer, lung cancer, breast cancer, genital cancer, a hematopoietic organ tumor, a bone and soft tissue tumor, skin cancer, and a brain tumor.

[44] The antitumor effect enhancer according to [15], the antitumor agent according to [16], the pharmaceutical composition according to any one of [17], [30], and [31], the compound 1 or a salt thereof according to [18], the combination according to [19], the method according to any one of [20] to [22], the use according to any one of [23]

to [29], or the antitumor effect enhancer, the antitumor agent, the pharmaceutical composition, the compound 1 or a salt thereof, the combination, the method, or the use according to any one of [32] to [43], wherein the tumor is lung cancer, pancreatic cancer, colorectal cancer, or a brain tumor.

Advantageous Effects of Invention

The present disclosure provides a novel combination therapy that exhibits excellent antitumor effects, and that uses (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and an MEK inhibitor.

Brief Description of Drawings

Fig. 1: The combinational effects of compound 1 (described as "TAS-120") and selumetinib in A549 cells are shown. KRAS-mutated lung cancer cell line A549 was treated with dimethylsulfoxide (DMSO) or compound 1 (10 nM or 100 nM), or DMSO or selumetinib (0.1 pM, 1 pM, or 10 μM), individually or in combination. The cells were cultured for 10 days with replacement of the medium every 72 hours or 96 hours. The cell culture plates were then stained with crystal violet.

Fig. 2: KRAS-mutated lung cancer cell line A549 cells were treated with DMSO or compound 1 (10 nM, 30 nM, or 100 nM) in combination with DMSO or trametinib (0.3 nM, 1.0 nM, 3.0 nM, 10 nM, or 30 nM), DMSO or binimetinib (3 nM, 10 nM, 30 nM, 100 nM, or 300nM), or DMSO or cobimetinib (3 nM, 10 nM, 30 nM, 100 nM, or 300 nM). The cells were cultured for 10 days with replacement of the medium every 72 hours or 96 hours. The cell culture plates were then stained with crystal violet.

Fig. 3: KRAS-mutated lung cancer cell line LU99 cells were treated with DMSO or compound 1 (10 nM, 30 nM, or 100 nM) in combination with DMSO or trametinib (0.3 nM, 1.0 nM, 3.0 nM, 10 nM, or 30nM), DMSO or binimetinib (3 nM, 10 nM, 30 nM, 100 nM, or 300 nM), or DMSO or cobimetinib (3 nM, 10 nM, 30 nM, 100 nM, or 300 nM). The cells were cultured for 10 days with replacement of the medium every 72 hours or 96 hours. The cell culture plates were then stained with crystal violet.

Fig. 4: Pharmacokinetics data ("PK data" below) of compound 1 at a QD dose (everyday administration) and a QOD dose (administration once every two days) disclosed in AACR2018, R. Bahleda et.al, poster CT121.

Description of Embodiments

The present disclosure relates to providing a combination of a compound with FGFR inhibition activity with an MEK inhibitor (i.e., an antitumor agent containing a compound with FGFR inhibition activity administered in combination with an MEK inhibitor, an antitumor effect enhancer for MEK inhibitors containing a compound with FGFR inhibition activity, an antitumor agent with FGFR inhibition activity administered to a patient who has been treated with an MEK inhibitor), tumor treatment methods, use of these agents, use of the compound in the production of these agents, and combinations thereof.

In the present disclosure, (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one is a known disubstituted benzene alkynyl compound that has the structure of the following chem. 1. The free form of compound 1 is also referred to as "TAS-120" or "futibatinib." Compound 1 is disclosed as Example Compound 2 in PTL 3 and can be synthesized, for example, according to the production method disclosed in PTL 3. PTL 3 reports that compound 1 or a salt thereof has excellent FGFR inhibitory activity and inhibits tyrosine phosphorylation in the substrate peptide sequence of the receptor protein tyrosine kinases of FGFR1, FGFR3, and FGFR4. PTL 5 reports an administration method in which compound 1 or a salt thereof is administered on "an administration schedule of at least twice a week and a dosing interval of at least one day."

The daily dose of the (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one (compound 1) or a salt thereof disclosed in the present specification is on a free-form basis.

Chem. 1

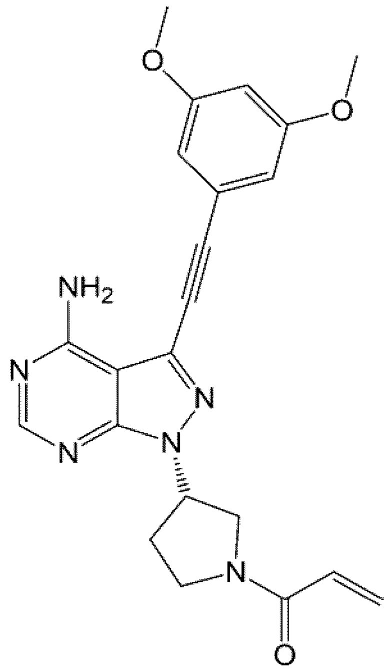

(I)

Although the scope of the MEK inhibitor is already common technical knowledge, the MEK inhibitor is not particularly limited in the present disclosure as long as the MEK inhibitor inhibits MEK1 and/or MEK2 in the MAPK/ERK signaling pathway that contributes to cell proliferation to bring about antitumor effects. Specifically, the MEK inhibitor includes trametinib, binimetinib, cobimetinib, selumetinib, pimasertib, mirdametinib, refametinib, NFX-179, HL-085, CS-3006, BI-3011441, FCN-159, E6201, TQ-B3234, SHR-7390, VS-6766, and pharmaceutically acceptable salts thereof. Of these, the MEK inhibitor is preferably trametinib, binimetinib, cobimetinib, selumetinib, or a pharmaceutically acceptable salt thereof, more preferably trametinib, binimetinib, cobimetinib, or a pharmaceutically acceptable salt thereof, and most preferably trametinib or a pharmaceutically acceptable salt thereof. In another embodiment, the MEK inhibitor is preferably trametinib dimethyl sulfoxide, binimetinib, or cobimetinib fumarate, and more preferably trametinib dimethyl sulfoxide.

In another embodiment, the MEK inhibitor is most preferably binimetinib or a pharmaceutically acceptable salt thereof. In another embodiment, the MEK inhibitor is more preferably binimetinib.

In another embodiment, the MEK inhibitor is most preferably cobimetinib or a pharmaceutically acceptable salt thereof. In another embodiment, the MEK inhibitor is more preferably cobimetinib fumarate. Examples of the pharmaceutically acceptable salts in these MEK inhibitors include, but are not particularly limited to, addition salts with inorganic acids such as hydrochloric acid or sulfuric acid, addition salts with organic acids such as acetic acid, citric acid, fumaric acid, tartaric acid, or maleic acid, salts with alkali metals such as potassium or sodium, salts with alkaline earth metals such as calcium or magnesium, and salts with organic bases such as ammonium salts, ethylamine salts, or arginine salts. The pharmaceutically acceptable salts of these compounds (MEK inhibitors) may be simply referred to as a salt of such a compound (e.g., a salt of trametinib).

Trametinib (chemical name: N-(3-{3-cyclopropyl-5-[(2-fluoro-4-iodophenyl)amino]-6,8-dimethyl-2,4,7-trioxo-3,4,6,7-tetrahydropyrido[4,3-d]pyrimidin-1(2H)-yl}phenyl)acetamide) is a known compound and has the following structure of chem. 2.

Chem. 2

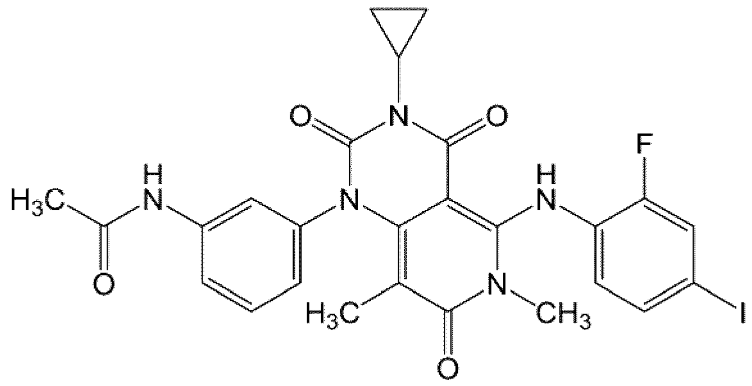

Trametinib can be synthesized according to the method disclosed in WO2005/121142. A commercial drug (Mekinist tablet (registered trademark), chemical name: N-(3-{3-cyclopropyl-5-[(2-fluoro-4-iodophenyl)amino]-6,8-dimethyl-2,4,7-trioxo-3,4,6,7-tetrahydropyrido[4,3-d]pyrimidin-1(2H)-yl}phenyl)acetamide-(methylsulfinyl)methane (1:1)) is also usable.

The daily dose of trametinib or a salt thereof described in the present specification is on a free-form basis.

Binimetinib (chemical name: 5-[(4-bromo-2-fluorophenyl)amino]-4-fluoro-N-(2-hydroxyethoxy)-1-methyl-1H-benzimidazole-6-carboxamide) is a known compound and has the following structure of chem. 3.

Chem. 3

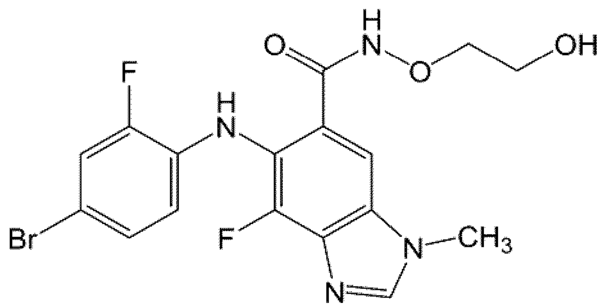

Binimetinib can be synthesized according to the method disclosed in WO2005/051906. A commercial drug (Mektovi tablet (registered trademark)) is also usable.

The daily dose of binimetinib or a salt thereof described in the present specification is on a free-form basis.

Cobimetinib (chemical name: [3,4-difluoro-2-[(2-fluoro-4-iodophenyl)amino]phenyl][3-hydroxy-3-[(2S)-2-piperidinyl]-1-azetidinyl]methanone) is a known compound and has the following structure of chem. 4.

Chem. 4

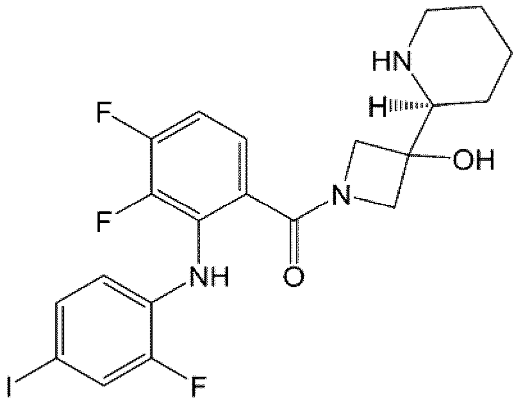

Cobimetinib can be synthesized according to the method disclosed in WO20007/044515. A commercial drug ((US brand name: Cotellic), or cobimetinib fumarate (chemical name: [3,4-difluoro-2-(2-fluoro-4-iodoanilino)phenyl]{3-hydroxy-3-[(2S)-piperidin-2-yl]azetidin-1-yl}methanone hemifumarate)) and other pharmaceutically acceptable salts are also usable.

The daily dose of cobimetinib or a salt thereof described in the present specification is on a free-form basis.

Selumetinib (chemical name: 5-[(4-bromo-2-chlorophenyl)amino]-4-fluoro-N-(2-hydroxyethoxy)-1-methyl-1H-benzimidazole-6-carboxamide) is a known compound and has the following structure of chem. 5.

Chem. 5

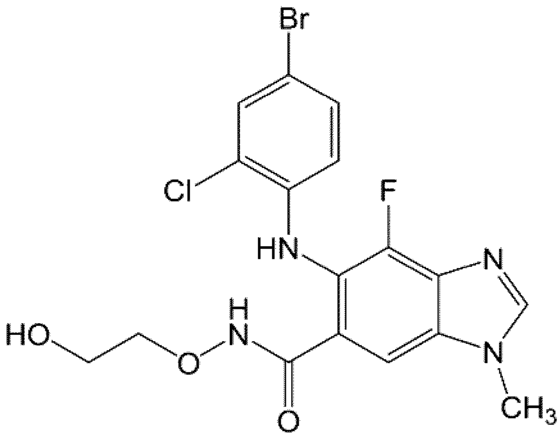

Selumetinib can be synthesized according to the method disclosed in WO2003/077914. Its commercially available products are also usable.

Pimasertib (chemical name: N-[(2S)-2,3-dihydroxypropyl]-3-[(2-fluoro-4-iodophenyl) amino]isonicotinamide) is a known compound and has the following structure of chem. 6.

Chem. 6

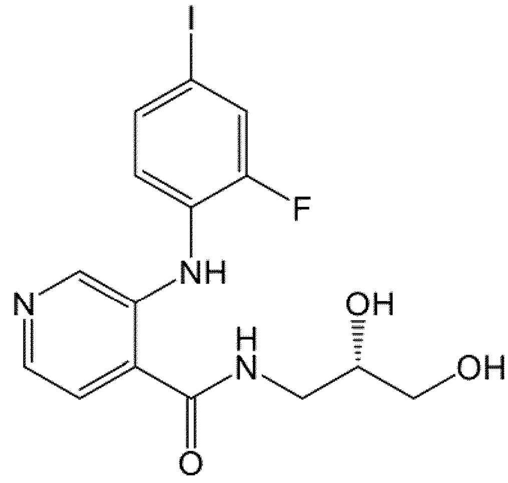

Pimasertib can be synthesized according to the method disclosed in WO2006/045514. Its commercially available products are also usable.

Mirdametinib (chemical name: (R)-N-(2,3-dihydroxypropoxy)-3,4-difluoro-2-((2-fluoro-4-iodophenyl)amino) benzamide) is a known compound and has the following structure of chem. 7.

Chem. 7

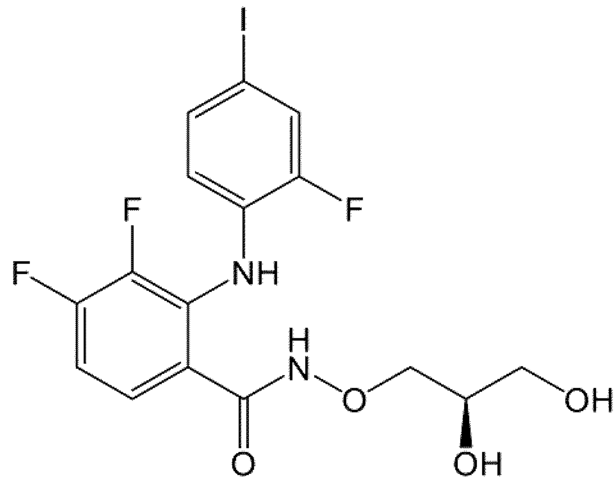

Mirdametinib can be synthesized according to the method disclosed in WO2002/006213. Its commercially available products are also usable.

Refametinib (chemical name: (R)-3-(1-((3,4-difluoro-2-((2-fluoro-4-iodophenyl)amino)-6-methoxybenzyl)sulfonyl) cyclopropyl)propane-1,2-diol) is a known compound and has the following structure of chem. 8.

Chem. 8

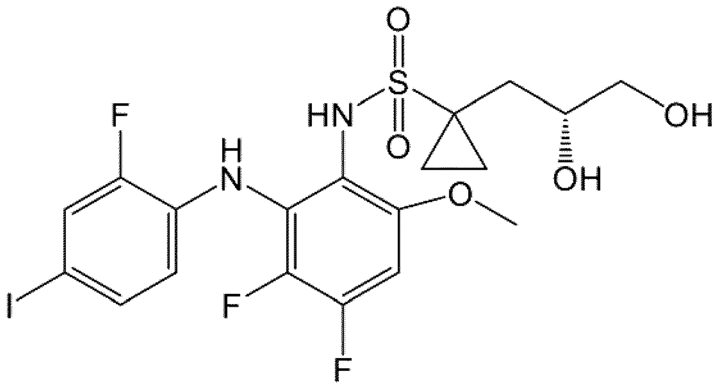

Refametinib can be synthesized according to the method disclosed in WO2007/014011. Its commercially available products are also usable.

In the present disclosure, the "recommended dose" refers to a dose that brings about the maximum therapeutic effect to the extent that the substance in question can be safely used without developing serious side effects, and that has been determined by clinical trials or like settings. Specific examples include doses that have been approved, recommended, or advised by public institutions or organizations, such as the Japan Pharmaceuticals and Medical Devices Agency (PMDA), the U.S. Food and Drug Administration (FDA), and the European Medicines Agency (EMA), and that are described in package insert, interview form, or treatment guideline. Doses approved by any of these public agencies (PMDA, FDA, and EMA) are preferable.

In the present disclosure, compound 1 can be used as is, or in the form of a pharmaceutically acceptable salt. Examples of pharmaceutically acceptable salts of compound 1 include, but are not particularly limited to, addition salts with inorganic acids such as hydrochloric acid or sulfuric acid, addition salts with organic acids such as acetic acid, citric acid, fumaric acid, tartaric acid, or maleic acid, salts with alkali metals such as potassium or sodium, salts with alkaline earth salts such as calcium or magnesium, and salts with organic bases such as ammonium salts, ethylamine salts, or arginine salts. In the present disclosure, a pharmaceutically acceptable salt of compound 1 may be simply referred to as a salt of compound 1.

In the present disclosure, compound 1 or a pharmaceutically acceptable salt thereof can be administered to humans and also other mammals (e.g., rats, mice, rabbits, sheep, swine, cows, cats, dogs, and monkeys). In the present disclosure, the MEK inhibitor can be administered to humans and also other mammals (e.g., rats, mice, rabbits, sheep, swine, cows, cats, dogs, and monkeys).

In the present disclosure, from the standpoint of the action of enhancing the antitumor effect of the MEK inhibitor by compound 1 or a salt thereof, when compound 1 or a salt thereof is administered every day, the preferable daily dose on the day of administration is preferably 40% to 120%, more preferably 40% to 100%, still more preferably 60 to 100%, yet more preferably 80 to 100%, and most preferably 100% of the recommended dose of compound 1 or a salt thereof administered alone every day. In another embodiment, when compound 1 or a salt thereof is administered every day, the preferable daily dose on the day of administration is 80% of the recommended dose of compound 1 or a salt thereof administered alone every day. In yet another embodiment, when compound 1 or a salt thereof is administered every day, the preferable daily dose on the day of administration is 60% of the recommended dose of compound 1 or a salt thereof administered alone every day.

Specifically, when compound 1 or a salt thereof is administered once a day for consecutive days, the preferable dose is, for example, 8 mg to 24 mg, 8 mg to 20 mg, 12 to 20 mg, or 20 mg. More specifically, the number of doses per day and the dose on the day of administration are, for example, once daily and 8 mg, 12 mg, 16 mg, 20 mg, or 24 mg. In another embodiment, the preferable number of doses per day and the preferable dose on the day of administration are, for example, once daily and 8 mg, 12 mg, 16 mg, or 20 mg. In another embodiment, the preferable number of doses per day and the preferable dose on the day of administration are, for example, once daily, and 12 mg, 16 mg, or 20 mg. In another embodiment, the preferable number of doses per day and the preferable dose on the day of administration are, for example, once daily and 20 mg. In another embodiment, the preferable number of doses per day and the preferable dose on the day of administration are, for example, once daily and 16 mg. In yet another embodiment, the preferable number of doses per day and the preferable dose on the day of administration are, for example, once daily and 12 mg. These doses of compound 1 or a salt thereof are all on a free-form basis.

In the present disclosure, from the standpoint of the action of enhancing the antitumor effect of the MEK inhibitor by compound 1 or a salt thereof, when compound 1 or a salt thereof is administered intermittently, the daily dose on the day of administration is preferably 15% to 125%, more preferably 35% to 100%, still more preferably 75 to 100%, preferably 80 to 100%, and most preferably 100% of the recommended dose of compound 1 or a salt thereof administered

alone intermittently.

Specifically, when compound 1 or a salt thereof is administered intermittently, the preferable daily dose on the day of administration is, for example, 24 mg to 200 mg. In another embodiment, the preferable daily dose on the day of administration is, for example, 56 mg to 160 mg. In another embodiment, the preferable daily dose on the day of administration is, for example, 120 to 160 mg. In another embodiment, the preferable daily dose on the day of administration is, for example, 160 mg. More specifically, the number of doses per day and the dose on the day of administration are, for example, once daily, and 24 mg, 36 mg, 56 mg, 80 mg, 120 mg, 160 mg, or 200 mg. In another embodiment, the preferable number of doses per day and the preferable dose on the day of administration are, for example, 56 mg, 80 mg, 120 mg, or 160 mg. In another embodiment, the preferable number of doses per day and the preferable dose on the day of administration are, for example, 80 mg, 120 mg, or 160 mg. In another embodiment, the preferable number of doses per day and the preferable dose on the day of administration are, for example, 160 mg. These doses of compound 1 or a salt thereof are all on a free-form basis.

In the present disclosure, from the standpoint of the action of enhancing the antitumor effect of the MEK inhibitor by compound 1 or a salt thereof, the daily dose of the MEK inhibitor on the day of administration is preferably 25% to 200%, more preferably 66 to 100%, and most preferably 100% of the recommended dose of the MEK inhibitor administered alone.

In the present disclosure, from the standpoint of the action of enhancing the antitumor effect of the MEK inhibitor by compound 1 or a salt thereof, the daily dose of trametinib or a salt thereof on the day of administration is preferably 25% to 200%, more preferably 50 to 100%, and most preferably 100% of the recommended dose of trametinib or a salt thereof administered alone. Specifically, the recommended dose of trametinib or a salt thereof administered alone to a human is 0.5 to 4 mg, more preferably 1 to 2 mg, and most preferably 2 mg when administered once daily, according to the approval information on the package insert or interview form. In an embodiment, the preferable number of doses per day and the preferable dose on the day of administration are, for example, once daily, and 0.5 mg, 1 mg, 1.5 mg, 2 mg, 3 mg, or 4 mg. In another embodiment, the preferable number of doses per day and the preferable dose on the day of administration are, for example, once daily, and 1 mg, 1.5 mg, or 2 mg. In another embodiment, the preferable number of doses per day and the preferable dose on the day of administration are, for example, once daily and 2 mg. These doses of trametinib or a salt thereof are all on a free-form basis.

In the present disclosure, from the standpoint of the action of enhancing the antitumor effect of the MEK inhibitor by compound 1 or a salt thereof, the daily dose of binimetinib or a salt thereof on the day of administration is preferably 66% to 133%, more preferably 66% to 100%, and most preferably 100% of the recommended dose of binimetinib or a salt thereof administered alone. Specifically, the recommended dose of binimetinib or a salt thereof administered alone to a human is 15 to 60 mg, more preferably 30 to 60 mg, more preferably 30 to 45 mg, and still more preferably 45 mg per dose when administered twice daily, according to the approval information on the package insert or interview form. In an embodiment, the preferable number of doses per day and the preferable dose on the day of administration are, for example, twice daily, and 15 mg, 30 mg, 45 mg, or 60 mg per dose. In another embodiment, the preferable number of doses per day and the preferable dose on the day of administration are, for example, twice daily, and 15 mg, 30 mg, or 45 mg per dose. In another embodiment, the preferable number of doses per day and the preferable dose on the day of administration are, for example, twice daily and 45 mg per dose. In another embodiment, the preferable number of doses per day and the preferable dose on the day of administration are, for example, twice daily and 30 mg per dose. In yet another embodiment, the preferable number of doses per day and the preferable dose on the day of administration are, for example, twice daily and 15 mg per dose. These doses of binimetinib or a salt thereof are all on a free-form basis.

In the present disclosure, from the standpoint of the action of enhancing the antitumor effect of the MEK inhibitor by compound 1 or a salt thereof, the daily dose of cobimetinib or a salt thereof on the day of administration is preferably 66% to 133%, more preferably 66% to 100%, and most preferably 100% of the recommended dose of cobimetinib or a salt thereof administered alone. Specifically, the recommended dose of cobimetinib or a salt thereof administered alone to a human is 40 to 80 mg, more preferably 40 to 60 mg, and most preferably 60 mg per dose when administered once daily, according to the approval information on the package insert or interview form. In an embodiment, the preferable number of doses per day and the preferable dose on the day of administration are, for example, once daily, and 40 mg, 60 mg, or 80 mg. In another embodiment, the preferable number of doses per day and the preferable dose on the day of administration are, for example, once daily, and 40 mg or 60 mg. In another embodiment, the preferable number of doses per day and the preferable dose on the day of administration are, for example, once daily and 60 mg. These doses of cobimetinib or a salt thereof are all on a free-form basis.

In the present disclosure, from the standpoint of the action of enhancing the antitumor effect of the MEK inhibitor by compound 1 or a salt thereof, the daily dose of selumetinib or a salt thereof on the day of administration is preferably 66% to 133%, more preferably 66% to 100%, and most preferably 100% of the recommended dose of selumetinib or a salt thereof administered alone. Specifically, the recommended dose of selumetinib or a salt thereof administered alone to a human is 5 to 25 $mg/m^2$, more preferably 20 to 25 $mg/m^2$, and still more preferably 25 $mg/m^2$ per dose when administered twice daily, according to the approval information on the package insert or interview form. In an embodiment,

the preferable number of doses per day and the preferable dose on the day of administration are, for example, twice daily, and 75 mg, 70 mg, 65 mg, 60 mg, 55 mg, 50 mg, 45 mg, 40 mg, 35 mg, 30 mg, 25 mg, 20 mg, or 10 mg. These doses of selumetinib or a salt thereof are all on a free-form basis.

In the present disclosure, from the standpoint of the action of enhancing the antitumor effect of the MEK inhibitor by compound 1 or a salt thereof, the daily dose of pimasertib or a salt thereof on the day of administration is preferably 66% to 133%, more preferably 66% to 100%, and most preferably 100% of the recommended dose of pimasertib or a salt thereof administered alone. In an embodiment, the preferable number of doses per day and the preferable dose on the day of administration are, for example, twice daily, and 10 to 60 mg. In another embodiment, the preferable number of doses per day and the preferable dose on the day of administration are, for example, once daily, and 10 to 60 mg. In yet another embodiment, the preferable number of doses per day and the preferable dose on the day of administration are, for example, twice daily, and 90 mg, 75 mg, 60 mg, 42 mg, 30 mg, 23 mg, 15 mg, or 8 mg. These doses of pimasertib or a salt thereof are all on a free-form basis.

In the present disclosure, from the standpoint of the action of enhancing the antitumor effect of the MEK inhibitor by compound 1 or a salt thereof, the daily dose of mirdametinib or a salt thereof on the day of administration is preferably 66% to 133%, more preferably 66% to 100%, and most preferably 100% of the recommended dose of mirdametinib or a salt thereof administered alone. Specifically, the recommended dose of mirdametinib or a salt thereof administered alone to a human is 1 to 5 $mg/m^2$, and more preferably 2 to 4 $mg/m^2$ per dose when administered twice daily. These doses of mirdametinib or a salt thereof are all on a free-form basis.

In the present disclosure, from the standpoint of the action of enhancing the antitumor effect of the MEK inhibitor by compound 1 or a salt thereof, the daily dose of refametinib or a salt thereof on the day of administration is preferably 66% to 133%, more preferably 66% to 100%, and most preferably 100% of the recommended dose of refametinib or a salt thereof administered alone. Specifically, the recommended dose of refametinib or a salt thereof administered alone to a human is 60 mg, 50 mg, 40 mg, 30 mg, 20 mg, or 10 mg per day when administered once daily or twice daily. These doses of refametinib or a salt thereof are all on a free-form basis.

Examples of the daily dose of compound 1 or a salt thereof of the present disclosure on the day of administration, and the daily dose of trametinib or a salt thereof, or preferred combinations of usage and dosage include the following:

8 to 20 mg per dose once a day, for consecutive days, of compound 1 or a salt thereof of the present disclosure, and 1 to 2 mg per dose once a day of trametinib or a salt thereof;
8 to 20 mg per dose once a day, for consecutive days, of compound 1 or a salt thereof of the present disclosure, and 1.5 to 2 mg per dose once a day of trametinib or a salt thereof;
8 to 20 mg per dose once a day, for consecutive days, of compound 1 or a salt thereof of the present disclosure, and 1 mg per dose once a day of trametinib or a salt thereof;
8 to 20 mg per dose once a day, for consecutive days, of compound 1 or a salt thereof of the present disclosure, and 1.5 mg per dose once a day of trametinib or a salt thereof;
8 to 20 mg per dose once a day, for consecutive days, of compound 1 or a salt thereof of the present disclosure, and 2 mg per dose once a day of trametinib or a salt thereof;
12 to 20 mg per dose once a day, for consecutive days, of compound 1 or a salt thereof of the present disclosure, and 1 to 2 mg per dose once a day of trametinib or a salt thereof;
12 to 20 mg per dose once a day, for consecutive days, of compound 1 or a salt thereof of the present disclosure, and 1.5 to 2 mg per dose once a day of trametinib or a salt thereof;
12 to 20 mg per dose once a day, for consecutive days, of compound 1 or a salt thereof of the present disclosure, and 1 mg per dose once a day of trametinib or a salt thereof;
12 to 20 mg per dose once a day, for consecutive days, of compound 1 or a salt thereof of the present disclosure, and 1.5 mg per dose once a day of trametinib or a salt thereof;
12 to 20 mg per dose once a day, for consecutive days, of compound 1 or a salt thereof of the present disclosure, and 2 mg per dose once a day of trametinib or a salt thereof;
8 mg per dose once a day, for consecutive days, of compound 1 or a salt thereof of the present disclosure, and 1 to 2 mg per dose once a day of trametinib or a salt thereof;
8 mg per dose once a day, for consecutive days, of compound 1 or a salt thereof of the present disclosure, and 1.5 to 2 mg per dose once a day of trametinib or a salt thereof;
8 mg per dose once a day, for consecutive days, of compound 1 or a salt thereof of the present disclosure, and 1 mg per dose once a day of trametinib or a salt thereof;
8 mg per dose once a day, for consecutive days, of compound 1 or a salt thereof of the present disclosure, and 1.5 mg per dose once a day of trametinib or a salt thereof;
8 mg per dose once a day, for consecutive days, of compound 1 or a salt thereof of the present disclosure, and 2 mg per dose once a day of trametinib or a salt thereof;
12 mg per dose once a day, for consecutive days, of compound 1 or a salt thereof of the present disclosure, and 1

to 2 mg per dose once a day of trametinib or a salt thereof;

12 mg per dose once a day, for consecutive days, of compound 1 or a salt thereof of the present disclosure, and 1.5 to 2 mg per dose once a day of trametinib or a salt thereof;

12 mg per dose once a day, for consecutive days, of compound 1 or a salt thereof of the present disclosure, and 1 mg per dose once a day of trametinib or a salt thereof;

12 mg per dose once a day, for consecutive days, of compound 1 or a salt thereof of the present disclosure, and 1.5 mg per dose once a day of trametinib or a salt thereof;

12 mg per dose once a day, for consecutive days, of compound 1 or a salt thereof of the present disclosure, and 2 mg per dose once a day of trametinib or a salt thereof;

16 mg per dose once a day, for consecutive days, of compound 1 or a salt thereof of the present disclosure, and 1 to 2 mg per dose once a day of trametinib or a salt thereof;

16 mg per dose once a day, for consecutive days, of compound 1 or a salt thereof of the present disclosure, and 1.5 to 2 mg per dose once a day of trametinib or a salt thereof;

16 mg per dose once a day, for consecutive days, of compound 1 or a salt thereof of the present disclosure, and 1 mg per dose once a day of trametinib or a salt thereof;

16 mg per dose once a day, for consecutive days, of compound 1 or a salt thereof of the present disclosure, and 1.5 mg per dose once a day of trametinib or a salt thereof;

16 mg per dose once a day, for consecutive days, of compound 1 or a salt thereof of the present disclosure, and 2 mg per dose once a day of trametinib or a salt thereof;

20 mg per dose once a day, for consecutive days, of compound 1 or a salt thereof of the present disclosure, and 1 to 2 mg per dose once a day of trametinib or a salt thereof;

20 mg per dose once a day, for consecutive days, of compound 1 or a salt thereof of the present disclosure, and 1.5 to 2 mg per dose once a day of trametinib or a salt thereof;

20 mg per dose once a day, for consecutive days, of compound 1 or a salt thereof of the present disclosure, and 1 mg per dose once a day of trametinib or a salt thereof;

20 mg per dose once a day, for consecutive days, of compound 1 or a salt thereof of the present disclosure, and 1.5 mg per dose once a day of trametinib or a salt thereof; and

20 mg per dose once a day, for consecutive days, of compound 1 or a salt thereof of the present disclosure, and 2 mg per dose once a day of trametinib or a salt thereof.

Examples of the daily dose of compound 1 or a salt thereof of the present disclosure on the day of administration, and the daily dose of binimetinib or a salt thereof, or preferred combinations of usage and dosage include the following:

8 to 20 mg per dose once a day, for consecutive days, of compound 1 or a salt thereof of the present disclosure, and 30 to 60 mg per dose twice a day of binimetinib or a salt thereof;

8 to 20 mg per dose once a day, for consecutive days, of compound 1 or a salt thereof of the present disclosure, and 30 to 45 mg per dose twice a day of binimetinib or a salt thereof;

8 to 20 mg per dose once a day, for consecutive days, of compound 1 or a salt thereof of the present disclosure, and 30 mg per dose twice a day of binimetinib or a salt thereof;

8 to 20 mg per dose once a day, for consecutive days, of compound 1 or a salt thereof of the present disclosure, and 45 mg per dose twice a day of binimetinib or a salt thereof;

12 to 20 mg per dose once a day, for consecutive days, of compound 1 or a salt thereof of the present disclosure, and 30 to 60 mg per dose twice a day of binimetinib or a salt thereof;

12 to 20 mg per dose once a day, for consecutive days, of compound 1 or a salt thereof of the present disclosure, and 30 to 45 mg per dose twice a day of binimetinib or a salt thereof;

12 to 20 mg per dose once a day, for consecutive days, of compound 1 or a salt thereof of the present disclosure, and 30 mg per dose twice a day of binimetinib or a salt thereof;

12 to 20 mg per dose once a day, for consecutive days, of compound 1 or a salt thereof of the present disclosure, and 45 mg per dose twice a day of binimetinib or a salt thereof;

8 mg per dose once a day, for consecutive days, of compound 1 or a salt thereof of the present disclosure, and 30 to 60 mg per dose twice a day of binimetinib or a salt thereof;

8 mg per dose once a day, for consecutive days, of compound 1 or a salt thereof of the present disclosure, and 30 to 45 mg per dose twice a day of binimetinib or a salt thereof;

8 mg per dose once a day, for consecutive days, of compound 1 or a salt thereof of the present disclosure, and 30 mg per dose twice a day of binimetinib or a salt thereof;

8 mg per dose once a day, for consecutive days, of compound 1 or a salt thereof of the present disclosure, and 45 mg per dose twice a day of binimetinib or a salt thereof;

12 mg per dose once a day, for consecutive days, of compound 1 or a salt thereof of the present disclosure, and

30 to 60 mg per dose twice a day of binimetinib or a salt thereof;
12 mg per dose once a day, for consecutive days, of compound 1 or a salt thereof of the present disclosure, and 30 to 45 mg per dose twice a day of binimetinib or a salt thereof;
12 mg per dose once a day, for consecutive days, of compound 1 or a salt thereof of the present disclosure, and 30 mg per dose twice a day of binimetinib or a salt thereof;
12 mg per dose once a day, for consecutive days, of compound 1 or a salt thereof of the present disclosure, and 45 mg per dose twice a day of binimetinib or a salt thereof;
16 mg per dose once a day, for consecutive days, of compound 1 or a salt thereof of the present disclosure, and 30 to 60 mg per dose twice a day of binimetinib or a salt thereof;
16 mg per dose once a day, for consecutive days, of compound 1 or a salt thereof of the present disclosure, and 30 to 45 mg per dose twice a day of binimetinib or a salt thereof;
16 mg per dose once a day, for consecutive days, of compound 1 or a salt thereof of the present disclosure, and 30 mg per dose twice a day of binimetinib or a salt thereof;
16 mg per dose once a day, for consecutive days, of compound 1 or a salt thereof of the present disclosure, and 45 mg per dose twice a day of binimetinib or a salt thereof;
20 mg per dose once a day, for consecutive days, of compound 1 or a salt thereof of the present disclosure, and 30 to 60 mg per dose twice a day of binimetinib or a salt thereof;
20 mg per dose once a day, for consecutive days, of compound 1 or a salt thereof of the present disclosure, and 30 to 45 mg per dose twice a day of binimetinib or a salt thereof;
20 mg per dose once a day, for consecutive days, of compound 1 or a salt thereof of the present disclosure, and 30 mg per dose twice a day of binimetinib or a salt thereof; and
20 mg per dose once a day, for consecutive days, of compound 1 or a salt thereof of the present disclosure, and 45 mg per dose twice a day of binimetinib or a salt thereof.

Examples of the daily dose of compound 1 or a salt thereof of the present disclosure on the day of administration, and the daily dose of cobimetinib or a salt thereof, or preferred combinations of usage and dosage include the following:

8 to 20 mg per dose once a day, for consecutive days, of compound 1 or a salt thereof of the present disclosure, and 40 to 80 mg per dose once a day of cobimetinib or a salt thereof;
8 to 20 mg per dose once a day, for consecutive days, of compound 1 or a salt thereof of the present disclosure, and 40 to 60 mg per dose once a day of cobimetinib or a salt thereof;
8 to 20 mg per dose once a day, for consecutive days, of compound 1 or a salt thereof of the present disclosure, and 40 per dose once a day of cobimetinib or a salt thereof;
8 to 20 mg per dose once a day, for consecutive days, of compound 1 or a salt thereof of the present disclosure, and 60 mg per dose once a day of cobimetinib or a salt thereof;
12 to 20 mg per dose once a day, for consecutive days, of compound 1 or a salt thereof of the present disclosure, and 40 to 80 mg per dose once a day of cobimetinib or a salt thereof;
12 to 20 mg per dose once a day, for consecutive days, of compound 1 or a salt thereof of the present disclosure, and 40 to 60 mg per dose once a day of cobimetinib or a salt thereof;
12 to 20 mg per dose once a day, for consecutive days, of compound 1 or a salt thereof of the present disclosure, and 40 per dose once a day of cobimetinib or a salt thereof;
12 to 20 mg per dose once a day, for consecutive days, of compound 1 or a salt thereof of the present disclosure, and 60 mg per dose once a day of cobimetinib or a salt thereof;
8 mg per dose once a day, for consecutive days, of compound 1 or a salt thereof of the present disclosure, and 40 to 80 mg per dose once a day of cobimetinib or a salt thereof;
8 mg per dose once a day, for consecutive days, of compound 1 or a salt thereof of the present disclosure, and 40 to 60 mg per dose once a day of cobimetinib or a salt thereof;
8 mg per dose once a day, for consecutive days, of compound 1 or a salt thereof of the present disclosure, and 40 per dose once a day of cobimetinib or a salt thereof;
8 mg per dose once a day, for consecutive days, of compound 1 or a salt thereof of the present disclosure, and 60 mg per dose once a day of cobimetinib or a salt thereof;
12 mg per dose once a day, for consecutive days, of compound 1 or a salt thereof of the present disclosure, and 40 to 80 mg per dose once a day of cobimetinib or a salt thereof;
12 mg per dose once a day, for consecutive days, of compound 1 or a salt thereof of the present disclosure, and 40 to 60 mg per dose once a day of cobimetinib or a salt thereof;
12 mg per dose once a day, for consecutive days, of compound 1 or a salt thereof of the present disclosure, and 40 per dose once a day of cobimetinib or a salt thereof;
12 mg per dose once a day, for consecutive days, of compound 1 or a salt thereof of the present disclosure, and

60 mg per dose once a day of cobimetinib or a salt thereof;
16 mg per dose once a day, for consecutive days, of compound 1 or a salt thereof of the present disclosure, and 40 to 80 mg per dose once a day of cobimetinib or a salt thereof;
16 mg per dose once a day, for consecutive days, of compound 1 or a salt thereof of the present disclosure, and 40 to 60 mg per dose once a day of cobimetinib or a salt thereof;
16 mg per dose once a day, for consecutive days, of compound 1 or a salt thereof of the present disclosure, and 40 per dose once a day of cobimetinib or a salt thereof;
16 mg per dose once a day, for consecutive days, of compound 1 or a salt thereof of the present disclosure, and 60 mg per dose once a day of cobimetinib or a salt thereof;
20 mg per dose once a day, for consecutive days, of compound 1 or a salt thereof of the present disclosure, and 40 to 80 mg per dose once a day of cobimetinib or a salt thereof;
20 mg per dose once a day, for consecutive days, of compound 1 or a salt thereof of the present disclosure, and 40 to 60 mg per dose once a day of cobimetinib or a salt thereof;
20 mg per dose once a day, for consecutive days, of compound 1 or a salt thereof of the present disclosure, and 40 per dose once a day of cobimetinib or a salt thereof; and
20 mg per dose once a day, for consecutive days, of compound 1 or a salt thereof of the present disclosure, and 60 mg per dose once a day of cobimetinib or a salt thereof.

In another embodiment, examples of the daily dose of compound 1 or a salt thereof of the present disclosure on the day of administration, and the daily dose of trametinib or a salt thereof, or preferred combinations of usage and dosage include the following:

24 to 200 mg per dose for intermittent administration of compound 1 or a salt thereof of the present disclosure, and 1 to 2 mg per dose once a day, for consecutive days, of trametinib or a salt thereof;
24 to 200 mg per dose for intermittent administration of compound 1 or a salt thereof of the present disclosure, and 1.5 to 2 mg per dose once a day, for consecutive days, of trametinib or a salt thereof;
24 to 200 mg per dose for intermittent administration of compound 1 or a salt thereof of the present disclosure, and 1 mg per dose once a day, for consecutive days, of trametinib or a salt thereof;
24 to 200 mg per dose for intermittent administration of compound 1 or a salt thereof of the present disclosure, and 1.5 mg per dose once a day, for consecutive days, of trametinib or a salt thereof;
24 to 200 mg per dose for intermittent administration of compound 1 or a salt thereof of the present disclosure, and 2 mg per dose once a day, for consecutive days, of trametinib or a salt thereof;
56 to 160 mg per dose for intermittent administration of compound 1 or a salt thereof of the present disclosure, and 1 to 2 mg per dose once a day, for consecutive days, of trametinib or a salt thereof;
56 to 160 mg per dose for intermittent administration of compound 1 or a salt thereof of the present disclosure, and 1.5 to 2 mg per dose once a day, for consecutive days, of trametinib or a salt thereof;
56 to 160 mg per dose for intermittent administration of compound 1 or a salt thereof of the present disclosure, and 1 mg per dose once a day, for consecutive days, of trametinib or a salt thereof;
56 to 160 mg per dose for intermittent administration of compound 1 or a salt thereof of the present disclosure, and 1.5 mg per dose once a day, for consecutive days, of trametinib or a salt thereof;
56 to 160 mg per dose for intermittent administration of compound 1 or a salt thereof of the present disclosure, and 2 mg per dose once a day, for consecutive days, of trametinib or a salt thereof;
80 to 160 mg per dose for intermittent administration of compound 1 or a salt thereof of the present disclosure, and 1 to 2 mg per dose once a day, for consecutive days, of trametinib or a salt thereof;
80 to 160 mg per dose for intermittent administration of compound 1 or a salt thereof of the present disclosure, and 1.5 to 2 mg per dose once a day, for consecutive days, of trametinib or a salt thereof;
80 to 160 mg per dose for intermittent administration of compound 1 or a salt thereof of the present disclosure, and 1 mg per dose once a day, for consecutive days, of trametinib or a salt thereof;
80 to 160 mg per dose for intermittent administration of compound 1 or a salt thereof of the present disclosure, and 1.5 mg per dose once a day, for consecutive days, of trametinib or a salt thereof;
80 to 160 mg per dose for intermittent administration of compound 1 or a salt thereof of the present disclosure, and 2 mg per dose once a day, for consecutive days, of trametinib or a salt thereof;
56 mg per dose for intermittent administration of compound 1 or a salt thereof of the present disclosure, and 1 to 2 mg per dose once a day, for consecutive days, of trametinib or a salt thereof;
56 mg per dose for intermittent administration of compound 1 or a salt thereof of the present disclosure, and 1.5 to 2 mg per dose once a day, for consecutive days, of trametinib or a salt thereof;
56 mg per dose for intermittent administration of compound 1 or a salt thereof of the present disclosure, and 1 mg per dose once a day, for consecutive days, of trametinib or a salt thereof;

56 mg per dose for intermittent administration of compound 1 or a salt thereof of the present disclosure, and 1.5 mg per dose once a day, for consecutive days, of trametinib or a salt thereof;
56 mg per dose for intermittent administration of compound 1 or a salt thereof of the present disclosure, and 2 mg per dose once a day, for consecutive days, of trametinib or a salt thereof;
80 mg per dose for intermittent administration of compound 1 or a salt thereof of the present disclosure, and 1 to 2 mg per dose once a day, for consecutive days, of trametinib or a salt thereof;
80 mg per dose for intermittent administration of compound 1 or a salt thereof of the present disclosure, and 1.5 to 2 mg per dose once a day, for consecutive days, of trametinib or a salt thereof;
80 mg per dose for intermittent administration of compound 1 or a salt thereof of the present disclosure, and 1 mg per dose once a day, for consecutive days, of trametinib or a salt thereof;
80 mg per dose for intermittent administration of compound 1 or a salt thereof of the present disclosure, and 1.5 mg per dose once a day, for consecutive days, of trametinib or a salt thereof;
80 mg per dose for intermittent administration of compound 1 or a salt thereof of the present disclosure, and 2 mg per dose once a day, for consecutive days, of trametinib or a salt thereof;
120 mg per dose for intermittent administration of compound 1 or a salt thereof of the present disclosure, and 1 to 2 mg per dose once a day, for consecutive days, of trametinib or a salt thereof;
120 mg per dose for intermittent administration of compound 1 or a salt thereof of the present disclosure, and 1.5 to 2 mg per dose once a day, for consecutive days, of trametinib or a salt thereof;
120 mg per dose for intermittent administration of compound 1 or a salt thereof of the present disclosure, and 1 mg per dose once a day, for consecutive days, of trametinib or a salt thereof;
120 mg per dose for intermittent administration of compound 1 or a salt thereof of the present disclosure, and 1.5 mg per dose once a day, for consecutive days, of trametinib or a salt thereof;
120 mg per dose for intermittent administration of compound 1 or a salt thereof of the present disclosure, and 2 mg per dose once a day, for consecutive days, of trametinib or a salt thereof;
160 mg per dose for intermittent administration of compound 1 or a salt thereof of the present disclosure, and 1 to 2 mg per dose once a day, for consecutive days, of trametinib or a salt thereof;
160 mg per dose for intermittent administration of compound 1 or a salt thereof of the present disclosure, and 1.5 to 2 mg per dose once a day, for consecutive days, of trametinib or a salt thereof;
160 mg per dose for intermittent administration of compound 1 or a salt thereof of the present disclosure, and 1 mg per dose once a day, for consecutive days, of trametinib or a salt thereof;
160 mg per dose for intermittent administration of compound 1 or a salt thereof of the present disclosure, and 1.5 mg per dose once a day, for consecutive days, of trametinib or a salt thereof; and
160 mg per dose for intermittent administration of compound 1 or a salt thereof of the present disclosure, and 2 mg per dose once a day, for consecutive days, of trametinib or a salt thereof.

In another embodiment, examples of the daily dose of compound 1 or a salt thereof of the present disclosure on the day of administration, and the daily dose of binimetinib or a salt thereof, or preferred combinations of usage and dosage include the following:

24 to 200 mg per dose for intermittent administration of compound 1 or a salt thereof of the present disclosure, and 30 to 60 mg per dose twice a day, for consecutive days, of binimetinib or a salt thereof;
24 to 200 mg per dose for intermittent administration of compound 1 or a salt thereof of the present disclosure, and 30 to 45 mg per dose twice a day, for consecutive days, of binimetinib or a salt thereof;
24 to 200 mg per dose for intermittent administration of compound 1 or a salt thereof of the present disclosure, and 30 mg per dose twice a day, for consecutive days, of binimetinib or a salt thereof;
24 to 200 mg per dose for intermittent administration of compound 1 or a salt thereof of the present disclosure, and 45 mg per dose twice a day, for consecutive days, of binimetinib or a salt thereof;
56 to 160 mg per dose for intermittent administration of compound 1 or a salt thereof of the present disclosure, and 30 to 60 mg per dose twice a day, for consecutive days, of binimetinib or a salt thereof;
56 to 160 mg per dose for intermittent administration of compound 1 or a salt thereof of the present disclosure, and 30 to 45 mg per dose twice a day, for consecutive days, of binimetinib or a salt thereof;
56 to 160 mg per dose for intermittent administration of compound 1 or a salt thereof of the present disclosure, and 30 mg per dose twice a day, for consecutive days, of binimetinib or a salt thereof;
56 to 160 mg per dose for intermittent administration of compound 1 or a salt thereof of the present disclosure, and 45 mg per dose twice a day, for consecutive days, of binimetinib or a salt thereof;
80 to 160 mg per dose for intermittent administration of compound 1 or a salt thereof of the present disclosure, and 30 to 60 mg per dose twice a day, for consecutive days, of binimetinib or a salt thereof;
80 to 160 mg per dose for intermittent administration of compound 1 or a salt thereof of the present disclosure, and

30 to 45 mg per dose twice a day, for consecutive days, of binimetinib or a salt thereof;
80 to 160 mg per dose for intermittent administration of compound 1 or a salt thereof of the present disclosure, and 30 mg per dose twice a day, for consecutive days, of binimetinib or a salt thereof;
80 to 160 mg per dose for intermittent administration of compound 1 or a salt thereof of the present disclosure, and 45 mg per dose twice a day, for consecutive days, of binimetinib or a salt thereof;
56 mg per dose for intermittent administration of compound 1 or a salt thereof of the present disclosure, and 30 to 60 mg per dose twice a day, for consecutive days, of binimetinib or a salt thereof;
56 mg per dose for intermittent administration of compound 1 or a salt thereof of the present disclosure, and 30 to 45 mg per dose twice a day, for consecutive days, of binimetinib or a salt thereof;
56 mg per dose for intermittent administration of compound 1 or a salt thereof of the present disclosure, and 30 mg per dose twice a day, for consecutive days, of binimetinib or a salt thereof;
56 mg per dose for intermittent administration of compound 1 or a salt thereof of the present disclosure, and 45 mg per dose twice a day, for consecutive days, of binimetinib or a salt thereof;
80 mg per dose for intermittent administration of compound 1 or a salt thereof of the present disclosure, and 30 to 60 mg per dose twice a day, for consecutive days, of binimetinib or a salt thereof;
80 mg per dose for intermittent administration of compound 1 or a salt thereof of the present disclosure, and 30 to 45 mg per dose twice a day, for consecutive days, of binimetinib or a salt thereof;
80 mg per dose for intermittent administration of compound 1 or a salt thereof of the present disclosure, and 30 mg per dose twice a day, for consecutive days, of binimetinib or a salt thereof;
80 mg per dose for intermittent administration of compound 1 or a salt thereof of the present disclosure, and 45 mg per dose twice a day, for consecutive days, of binimetinib or a salt thereof;
120 mg per dose for intermittent administration of compound 1 or a salt thereof of the present disclosure, and 30 to 60 mg per dose twice a day, for consecutive days, of binimetinib or a salt thereof;
120 mg per dose for intermittent administration of compound 1 or a salt thereof of the present disclosure, and 30 to 45 mg per dose twice a day, for consecutive days, of binimetinib or a salt thereof;
120 mg per dose for intermittent administration of compound 1 or a salt thereof of the present disclosure, and 30 mg per dose twice a day, for consecutive days, of binimetinib or a salt thereof;
120 mg per dose for intermittent administration of compound 1 or a salt thereof of the present disclosure, and 45 mg per dose twice a day, for consecutive days, of binimetinib or a salt thereof;
160 mg per dose for intermittent administration of compound 1 or a salt thereof of the present disclosure, and 30 to 60 mg per dose twice a day, for consecutive days, of binimetinib or a salt thereof;
160 mg per dose for intermittent administration of compound 1 or a salt thereof of the present disclosure, and 30 to 45 mg per dose twice a day, for consecutive days, of binimetinib or a salt thereof;
160 mg per dose for intermittent administration of compound 1 or a salt thereof of the present disclosure, and 30 mg per dose twice a day, for consecutive days, of binimetinib or a salt thereof; and
160 mg per dose for intermittent administration of compound 1 or a salt thereof of the present disclosure, and 45 mg per dose twice a day, for consecutive days, of binimetinib or a salt thereof.

In another embodiment, examples of the daily dose of compound 1 or a salt thereof of the present disclosure on the day of administration, and the daily dose of cobimetinib or a salt thereof, or preferred combinations of usage and dosage include the following:

24 to 200 mg per dose for intermittent administration of compound 1 or a salt thereof of the present disclosure, and 40 to 80 mg per dose once a day, for consecutive days, of cobimetinib or a salt thereof;
24 to 200 mg per dose for intermittent administration of compound 1 or a salt thereof of the present disclosure, and 40 mg per dose once a day, for consecutive days, of cobimetinib or a salt thereof;
24 to 200 mg per dose for intermittent administration of compound 1 or a salt thereof of the present disclosure, and 60 mg per dose once a day, for consecutive days, of cobimetinib or a salt thereof;
24 to 200 mg per dose for intermittent administration of compound 1 or a salt thereof of the present disclosure, and 40 to 60 mg per dose once a day, for consecutive days, of cobimetinib or a salt thereof;
56 to 160 mg per dose for intermittent administration of compound 1 or a salt thereof of the present disclosure, and 40 to 80 mg per dose once a day, for consecutive days, of cobimetinib or a salt thereof;
56 to 160 mg per dose for intermittent administration of compound 1 or a salt thereof of the present disclosure, and 40 mg per dose once a day, for consecutive days, of cobimetinib or a salt thereof;
56 to 160 mg per dose for intermittent administration of compound 1 or a salt thereof of the present disclosure, and 60 mg per dose once a day, for consecutive days, of cobimetinib or a salt thereof;
56 to 160 mg per dose for intermittent administration of compound 1 or a salt thereof of the present disclosure, and 40 to 60 mg per dose once a day, for consecutive days, of cobimetinib or a salt thereof;

80 to 160 mg per dose for intermittent administration of compound 1 or a salt thereof of the present disclosure, and 40 to 80 mg per dose once a day, for consecutive days, of cobimetinib or a salt thereof;
80 to 160 mg per dose for intermittent administration of compound 1 or a salt thereof of the present disclosure, and 40 mg per dose once a day, for consecutive days, of cobimetinib or a salt thereof;
80 to 160 mg per dose for intermittent administration of compound 1 or a salt thereof of the present disclosure, and 60 mg per dose once a day, for consecutive days, of cobimetinib or a salt thereof;
80 to 160 mg per dose for intermittent administration of compound 1 or a salt thereof of the present disclosure, and 40 to 60 mg per dose once a day, for consecutive days, of cobimetinib or a salt thereof;
56 mg per dose for intermittent administration of compound 1 or a salt thereof of the present disclosure, and 40 to 80 mg per dose once a day, for consecutive days, of cobimetinib or a salt thereof;
56 mg per dose for intermittent administration of compound 1 or a salt thereof of the present disclosure, and 40 mg per dose once a day, for consecutive days, of cobimetinib or a salt thereof;
56 mg per dose for intermittent administration of compound 1 or a salt thereof of the present disclosure, and 60 mg per dose once a day, for consecutive days, of cobimetinib or a salt thereof;
56 mg per dose for intermittent administration of compound 1 or a salt thereof of the present disclosure, and 40 to 60 mg per dose once a day, for consecutive days, of cobimetinib or a salt thereof;
80 mg per dose for intermittent administration of compound 1 or a salt thereof of the present disclosure, and 40 to 80 mg per dose once a day, for consecutive days, of cobimetinib or a salt thereof;
80 mg per dose for intermittent administration of compound 1 or a salt thereof of the present disclosure, and 40 mg per dose once a day, for consecutive days, of cobimetinib or a salt thereof;
80 mg per dose for intermittent administration of compound 1 or a salt thereof of the present disclosure, and 60 mg per dose once a day, for consecutive days, of cobimetinib or a salt thereof;
80 mg per dose for intermittent administration of compound 1 or a salt thereof of the present disclosure, and 40 to 60 mg per dose once a day, for consecutive days, of cobimetinib or a salt thereof;
120 mg per dose for intermittent administration of compound 1 or a salt thereof of the present disclosure, and 40 to 80 mg per dose once a day, for consecutive days, of cobimetinib or a salt thereof;
120 mg per dose for intermittent administration of compound 1 or a salt thereof of the present disclosure, and 40 mg per dose once a day, for consecutive days, of cobimetinib or a salt thereof;
120 mg per dose for intermittent administration of compound 1 or a salt thereof of the present disclosure, and 60 mg per dose once a day, for consecutive days, of cobimetinib or a salt thereof;
120 mg per dose for intermittent administration of compound 1 or a salt thereof of the present disclosure, and 40 to 60 mg per dose once a day, for consecutive days, of cobimetinib or a salt thereof;
160 mg per dose for intermittent administration of compound 1 or a salt thereof of the present disclosure, and 40 to 80 mg per dose once a day, for consecutive days, of cobimetinib or a salt thereof;
160 mg per dose for intermittent administration of compound 1 or a salt thereof of the present disclosure, and 40 mg per dose once a day, for consecutive days, of cobimetinib or a salt thereof;
160 mg per dose for intermittent administration of compound 1 or a salt thereof of the present disclosure, and 60 mg per dose once a day, for consecutive days, of cobimetinib or a salt thereof; and
160 mg per dose for intermittent administration of compound 1 or a salt thereof of the present disclosure, and 40 to 60 mg per dose once a day, for consecutive days, of cobimetinib or a salt thereof.

In an embodiment of the present disclosure, in the case of a cancer type for which a stronger effect is desired (e.g., brain tumor), the dose of compound 1 or a salt thereof includes a dose of more than 20 mg once daily. The dose of trametinib or a salt thereof includes a dose of more than 2 mg once daily. The dose of binimetinib or a salt thereof includes a dose of more than 45 mg per dose twice daily. The dose of cobimetinib or a salt thereof includes a dose of more than 60 mg per dose once daily.

The order of administration of compound 1 or a salt thereof and the MEK inhibitor may be appropriately selected according to the type of cancer, the stage of cancer, and the like; however, in one therapeutic regimen, either of them may be administered first, or both of them may be administered simultaneously.

The administration schedule of the antitumor agent of the present disclosure can be appropriately selected according to the type of cancer, the stage of cancer, and the like. The administration schedule of compound 1 or a pharmaceutically acceptable salt thereof is preferably administration every day (i.e., for consecutive days) or intermittently. The administration schedule of trametinib or a salt thereof is preferably administration once a day for consecutive days. The administration schedule of binimetinib or a salt thereof is preferably administration twice a day for consecutive days. The administration schedule of cobimetinib or a salt thereof is preferably administration once a day for consecutive days. The administration schedule of selumetinib or a salt thereof is preferably administration twice a day for consecutive days. The administration schedule may be repeated. Further, a drug holiday may be appropriately provided depending on side effects and other factors.

In the present specification, "combined administration" or "administered in combination" is synonymous with administration in treatment in one therapeutic regimen.

In the present specification, "administered every day" may refer to, for example, an administration schedule in which a schedule of performing administration consecutively within one therapeutic regimen is taken as one cycle. For example, a 21-day therapeutic regimen refers to performing administration for 21 days every day. A drug holiday may be provided as each cycle ends.

In the present disclosure, "administered intermittently" is not particularly limited as long as the conditions of administration of at least twice a week and an administration interval (the number of days between a specific day of administration and the next day of administration) of at least one day are satisfied. It is preferred to perform administration at least three times a week, and it is more preferred to perform administration three times a week.

Examples include an administration schedule based on a 1-week cycle in which compound 1 or a pharmaceutically acceptable salt thereof is administered at least twice every one to three days per cycle (with an interval between a specific day of administration and the next day of administration of 1 to 3 days), and this cycle is performed once, or twice or more repeatedly;

an administration schedule based on a 14-day cycle in which compound 1 or a pharmaceutically acceptable salt thereof is administered 4 to 7 times every one to three days per cycle (with an interval between a specific day of administration and the next day of administration of 1 to 3 days), and this cycle is performed once, or twice or more repeatedly;
an administration schedule based on a 14-day cycle in which, among 14 days included in one cycle, compound 1 or a pharmaceutically acceptable salt thereof is administered on days 1, 4, 8, and 11;
an administration schedule based on a 14-day cycle in which, among 14 days included in one cycle, compound 1 or a pharmaceutically acceptable salt thereof is administered on days 1, 3, 5, 7, 9, 11, and 13; and
an administration schedule based on a 14-day cycle in which, among 14 days included in one cycle, compound 1 or a pharmaceutically acceptable salt thereof is administered on days 1, 3, 5, 8, 10, and 12.

In the present specification, the administration of drug A at intervals of X days means that when the day on which drug A is administered is day 1, the next day is day 2, and the day after is day 3, etc., drug A will be next administered on day X + 2.

The therapeutically effective amounts of compound 1 or a salt thereof and the MEK inhibitor are not limited, and depend on several factors, including the stage and severity of cancer, as well as other factors related to the patient's health. Those skilled in the art would know how to determine the therapeutically effective amounts.

The method of combination therapy described in the present specification is useful in the treatment of proliferative diseases. This method requires administration of effective amounts of compound 1 or a salt thereof and an MEK drug. In some embodiments, an effective amount refers to an amount sufficient to delay tumor growth. In another embodiment, an effective amount refers to an amount sufficient to prevent or delay recurrence. An effective amount may be administered in one or more doses.

For cancer, the effective amount of a drug or composition is an amount that can cause the following.

(i) Reduce the number of cancer cells.
(ii) Reduce the tumor size.
(iii) Reduce the burden from a tumor.
(iv) Suppress, delay, delay to some extent, and preferably stop the invasion of cancer cells into peripheral organs.
(v) Inhibit (i.e., delay to some extent, preferably stop) tumor metastasis.
(vi) Inhibit tumor growth.
(vii) Reduce the rate of tumor growth.
(viii) Prevent or delay tumor development and/or recurrence.
(ix) Alleviate at least one cancer-related symptom to some extent.
(x) Prolong life, and/or
(xi) extend progression-free survival.

The number of daily doses of the antitumor agent of the present disclosure can be appropriately selected according to the type of cancer, the stage of cancer, and the like. When compound 1 or a salt thereof is administered in combination with the MEK inhibitor, it is preferred that compound 1 or a salt thereof be administered once daily, trametinib or a salt thereof once daily, binimetinib or a salt thereof twice daily, cobimetinib or a salt thereof once daily, and selumetinib or a salt thereof twice daily.

The treatment in the present disclosure includes procedures performed for the purpose of curing or ameliorating diseases, or for the purpose of suppressing disease progression or relapse or alleviating the symptoms. The treatment

includes administration of drugs before or after a surgical procedure, or administration of drugs before, during, or after radiation therapy.

As used in the present specification, the term "combination (therapy)" is intended to define a therapy that includes the use of a combination of two or more compounds/drugs (as defined above). Thus, "combination (therapy)," "combination," and the use of compounds/drugs "in combination" in the present application may mean compounds/drugs administered as part of the same overall therapeutic regimen.

The term "regimen" or "therapeutic regimen" refers to a plan that shows, in chronological order, the type and amount of drugs, duration, procedure, etc. in drug treatment. Treatment in one therapeutic regimen means administration of a specific drug and another drug within a determined period of time according to administration intervals and doses prescribed for each drug. The treatment in one therapeutic regimen refers to, for example, a method that starts administration of drugs to be combined simultaneously or substantially simultaneously on the first day of the cycle. The treatment in one therapeutic regimen also includes, for example, one cycle with three weeks in which administration of drug A is started first, and administration of drug B is started one week later, and in which both drugs are substantially simultaneously administered in one cycle. The "determined period of time" may be, for example, 28 days, and can be selected according to the type of cancer, the stage of cancer, etc.

Before and/or after the therapeutic regimen starts, a washout period, i.e., a period of time when no medication is given, may be provided to eliminate the effects of previously administered drugs.

The therapeutic regimen of compound 1 or a salt thereof of the present disclosure and the MEK inhibitor can include not only compound 1 or a salt thereof and the MEK inhibitor, but also other drugs.

The expressions "used before," "used simultaneously," and "used after" refer to the order of administration of compound 1 or a pharmaceutically acceptable salt thereof and the MEK inhibitor on the same scheduled date of administration. The expression "used before" has the same meaning as that compound 1 or a salt thereof is administered before the MEK inhibitor on the same scheduled date of administration. The expression "used simultaneously" has the same meaning as that compound 1 or a salt thereof is administered simultaneously with the MEK inhibitor on the same scheduled date of administration. The expression "used after" has the same meaning as that compound 1 or a salt thereof is administered after the MEK inhibitor on the same scheduled date of administration.

The order of administration of compound 1 or a salt thereof and the MEK inhibitor may be appropriately selected according to the type of cancer, the stage of cancer, and the like; however, in one therapeutic regimen, either of them may be administered first, or both of them may be administered simultaneously.

In one embodiment, the combination therapy of the present disclosure is administered to patients who have not been treated with hormonal therapy, immunotherapy (cancer peptide vaccine therapy etc.), surgery, radiation therapy, or treatment with chemotherapeutic agents, i.e., treatment-naive patients. In another embodiment, the combination therapy is administered to patients who fail to achieve a sustained response after previous treatment with chemotherapeutic agents.

In the present disclosure, "cancer" or "tumor" refers to the physiological conditions of a mammal characterized by unregulated cell growth. "Cancer" and "tumor" have the same meaning in the present specification and are used interchangeably. Cancers include solid and hematologic cancers. Examples include, but are not limited to, carcinoma, lymphoma, leukemia, blastoma, sarcoma, and borderline malignancy (carcinoid).

In an embodiment of the present disclosure, diseases (in particular, tumors) involved in a Ras/MAPK signaling pathway can be prevented and/or treated.

Examples of the "diseases involved in a Ras/MAPK signaling pathway" include, but are not limited to, cancers, autoimmune diseases, and macroglobulinemia.

Typical examples of Ras/MAPK signaling pathways include, but are not particularly limited to, signaling such as Ras and downstream signaling cascades of Ras, such as RAF, MEK, ERK, PI3K, AKT, mTOR, and RAL-GEF. The diseases may be diseases (in particular, tumors) whose incidence can be reduced, whose symptoms can be ameliorated or alleviated, whose progression is suppressed, whose relapse is suppressed, and/or whose symptoms are completely cured, by deleting, suppressing, and/or inhibiting the functions of those pathways.

The diseases involved in a Ras/MAPK signaling pathway are preferably tumors having aberrations in Ras/MAPK signaling pathways, more preferably tumors having, for example, Ras overexpression, Ras genetic aberrations, aberrant Ras/MAPK signaling activity state, etc.

Tumors having Ras overexpression are preferably tumors having gene expression and high expression of gene product proteins.

Preferable examples of tumors having Ras genetic aberrations include those having gene amplification, gene mutation, chromosomal translocation, insertion, and inversion, gene fusion, and gene rearrangement. The phrase "tumors having a gene mutation" is preferably used for tumors having Ras genetic aberrations, more preferably for tumors having a mutation in an isoform of NRAS, KRAS, or HRAS, and still more preferably for tumors having a mutation in an isoform of KRAS.

Preferable examples of tumors having an aberrant Ras/MAPK signaling activity state include tumors having

increased activity of Ras or MEK.

The tumors having aberration in Ras/MAPK signaling pathways are more preferably tumors having Ras overexpression, Ras genetic aberrations, aberrant Ras/MAPK signaling activity state, etc., and still more preferably tumors having Ras genetic aberrations, and most preferably tumors having a mutation in an isoform of KRAS.

In another embodiment of the present disclosure, diseases involved in a Ras/MAPK signaling pathway are preferably tumors having aberrations in Ras/MAPK signaling pathways, and more preferably tumors having FGFR genetic aberrations.

The phrase "tumors having FGFR genetic aberrations" is preferably used for, for example, tumors having gene amplification, gene mutation, chromosomal translocation, insertion, and inversion, gene fusion, gene reconstitution, etc. The phrase is preferably used for tumors having a FGFR mutation, and more preferably tumors having a FGFR mutation selected from the group consisting of FGFR1, FGFR2, FGFR3, and FGFR4.

An embodiment of the present disclosure is used for tumors having Ras and/or FGFR overexpression. The expression of Ras and/or FGFR can be detected by methods known to those skilled in the art. Gene expression and high expression of gene product proteins can be detected by known methods, such as methods using antibodies (immunohistochemistry, enzyme immunoassay (ELISA), flow cytometry, immunoblotting, etc.), methods using nucleic acids (in situ hybridization, PCR, Northern blotting, etc.), and methods based on a common principle of these methods. The detection device can be any known device (GeneChip, microarray, etc.).

An embodiment of the present disclosure is used for tumors having Ras and/or FGFR genetic aberrations. Some of the Ras and/or FGFR genetic aberrations in tumors have already been reported in documents available to those skilled in the art (Non-patent Literature). Ras and/or FGFR genetic aberrations can be detected by methods known to those skilled in the art, such as pyrosequence, DNA sequencing including next-generation sequencing (NGS), PCR-based methods including allele-specific PCR chain reactions, microarray-based comparative genomic hybridization (aCGH), fluorescence in situ hybridization (FISH), and chromogenic in situ hybridization (CISH).

Further, an embodiment of the present disclosure is used for tumors with activated Ras signaling and/or FGFR signaling. Activation of Ras signaling can be detected by methods known to those skilled in the art. Signaling activation can be detected, for example, by detecting the direct GTP- or GDP-binding state of Ras, phosphorylation state of intracellular substrates and intracellular proteins present in the Ras signaling cascade downstream, or biological activity including enzymatic activity, or by detecting gene products or gene transcripts. Activation of FGFR signaling can be detected by methods known to those skilled in the art. Signaling activation can be detected, for example, by detecting FGFs, which are ligands for FGFRs, by detecting the direct phosphorylation state of FGFRs, which are phosphorylated enzymes, or biological activity including enzymatic activity, by detecting the phosphorylation state of intracellular substrates and intracellular proteins present in the FGFR signaling cascade downstream, or biological activity including enzymatic activity, or by detecting gene products or gene transcripts.

Examples of tumors targeted in the present disclosure include, but are not particularly limited to, head and neck cancer, gastroenterological cancer (esophageal cancer, stomach cancer, duodenal cancer, liver cancer, biliary cancer (e.g., gallbladder and bile duct cancer), pancreatic cancer, colorectal cancer (e.g., colon cancer and rectal cancer), etc.), lung cancer (non-small-cell lung cancer, small-cell lung cancer, mesothelioma, etc.), breast cancer, genital cancer (ovarian cancer, uterine cancer (e.g., cervical cancer and endometrial cancer), etc.), urological cancer (kidney cancer, bladder cancer, prostate cancer, testicular tumor, etc.), hematopoietic organ tumor (leukemia, malignant lymphoma, multiple myeloma, etc.), bone and soft tissue tumor, skin cancer, brain tumor, neurofibromatosis, and thyroid cancer. Preferred are lung cancer, pancreatic cancer, and colorectal cancer.

The cancers mentioned herein include not only the primary lesion, but also cancer metastasized to other organs (e.g., liver). The antitumor agent of the present disclosure may also be used in postoperative adjuvant chemotherapy performed in order to prevent recurrence after surgical removal of the tumor, or may be used in preoperative adjuvant chemotherapy performed in advance in order to surgically remove the tumor.

Examples of methods for detecting the expression of the mechanism of action by FGFR inhibitors and the mechanism of action by MEK inhibitors include, but are not limited to, a method for examining the modulation of the tumor immune microenvironment (Cellular Physiology and Biochemistry, 33, pp. 633-645 (2014)).

The administration form of the antitumor agent of the present disclosure is not particularly limited for compound 1 or a pharmaceutically acceptable salt thereof, or for the MEK inhibitor, and can be appropriately selected depending on the therapeutic purpose. Specific examples include oral preparations (tablets, coated tablets, powders, granules, capsules, solutions, etc.), injections, suppositories, patches, and ointments. Examples of the administration form of compound 1 or a salt thereof include those listed above, and oral preparations etc. are preferred. Examples of the administration form of the MEK inhibitors also include those listed above, and oral preparations etc. are preferred.

According to the antitumor agent of the present disclosure, compound 1 or a salt thereof and the MEK inhibitors, which are active ingredients, may be directly used as an antitumor agent. Depending on the administration form thereof, a pharmaceutically acceptable carrier may be used to prepare a pharmaceutical composition by a generally known method. Examples of such carriers include various carriers commonly used in general drugs, such as excipients, binders,

disintegrants, lubricants, diluents, solubilizing agents, suspending agents, isotonizing agents, pH adjusters, buffers, stabilizers, coloring agents, and flavoring agents.

The antitumor agent of the present disclosure may be formulated into multiple dosage forms, or may be formulated into a single dosage form, based on the administration form and/or administration schedule of each active ingredient. Further, formulations may be produced and sold in a single package suitable for combined administration, or formulations may be produced and sold in separate packages. The same applies to the embodiments of the pharmaceutical composition. Accordingly, the "pharmaceutical composition comprising compound 1 or a salt thereof and an MEK inhibitor as active ingredients" includes a pharmaceutical composition prepared by separately formulating the active ingredients in multiple dosage forms, and a pharmaceutical composition prepared by formulating the active ingredients in a single dosage form. The pharmaceutical composition prepared by separately formulating the active ingredients in multiple dosage forms includes a pharmaceutical composition prepared by formulating formulations in a single package suitable for combined administration, and a pharmaceutical composition prepared by formulating formulations in separate packages.

In one embodiment, the present disclosure relates to a kit preparation comprising an antitumor agent comprising compound 1 or a salt thereof, and an instruction manual stating that compound 1 or a salt thereof is administered to cancer patients in combination with an MEK inhibitor. The "instruction manual" as used herein may be one stating the dose described above. Regardless of whether it is legally binding, an instruction manual that recommends the dose described above is preferable. Specific examples include package inserts and brochures. The kit preparation comprising an instruction manual may be one in which the instruction manual is printed on or attached to the package of the kit preparation, or may be one in which the instruction manual is enclosed together with an antitumor agent in the package of the kit preparation.

The present disclosure also relates to a tumor treatment method comprising administering compound 1 or a salt thereof in combination with an MEK inhibitor.

The present disclosure also relates to a method for enhancing an antitumor effect of an MEK inhibitor comprising administering an antitumor agent comprising an agent containing compound 1 or a salt thereof.

The present disclosure also relates to a tumor treatment method for the treatment of a cancer patient to whom an MEK inhibitor is administered, the method comprising administering an antitumor agent comprising an agent containing compound 1 or a salt thereof to the cancer patient.

The present disclosure also relates to an antitumor agent of the present disclosure for use in the treatment of a tumor.

The present disclosure also relates to an antitumor effect enhancer comprising compound 1 or a salt thereof for use in the enhancement of an antitumor effect.

The present disclosure also relates to an antitumor agent comprising an agent containing compound 1 or a salt thereof for use in the treatment of a cancer patient to whom an MEK inhibitor is administered.

The present disclosure also relates to use of an antitumor agent of the present disclosure in combination therapy with an MEK inhibitor in the treatment of a tumor.

The present disclosure also relates to use of an antitumor agent of the present disclosure in the enhancement of an antitumor effect of an MEK inhibitor

The present disclosure also relates to use of an agent containing compound 1 or a salt thereof in the treatment of a cancer patient to whom an MEK inhibitor is administered.

The present disclosure also relates to use of an antitumor agent of the present disclosure in combination therapy with an MEK inhibitor in the manufacture of a pharmaceutical preparation for treating a tumor.

The present disclosure also relates to use of an antitumor agent of the present disclosure in the manufacture of a pharmaceutical preparation for enhancing an antitumor effect of an MEK inhibitor.

The present disclosure also relates to use of an antitumor agent comprising an agent containing compound 1 or a salt thereof in the manufacture of a pharmaceutical preparation for treating a cancer patient to whom an MEK inhibitor is administered.

In one embodiment of the present disclosure, the administration is performed to patients with Ras mutations or aberrations in Ras/MAPK signaling pathways, preferably with tumors having Ras genetic aberrations, more preferably with tumors having a mutation in an isoform of NRAS, KRAS, or HRAS, and still more preferably with tumors having a mutation in an isoform of KRAS.

The antitumor agent of the present disclosure can be used for the treatment of cancer. The "antitumor effect," when referred to for a cancer patient who receives treatment with a therapeutic regimen, such as the combination therapy described in the present specification, represents at least one evaluation of, for example, PFS (progression-free survival), DCR (disease control rate), DOR (duration of response), OS (overall survival), ORR (objective response rate), DCR (disease control rate), PROs (patient-reported outcomes), and the like. In one embodiment, tumor evaluation for the combination therapy described in the present specification for solid tumors is based on the RECIST 1.1 criteria (response evaluation criteria in solid tumors), and the antitumor effect is expressed as SD (stable disease), PR (partial response),

CR (complete response), or PD (progressive disease). Tumor evaluation of brain tumors can be carried out with standard brain tumor MRI, including pre- and post-enhancement MRI, using a Gd-MRI (gadolinium (Gd)) chelate contrast agent.

Examples

The present disclosure is explained in more detail below with reference to Examples and Reference Examples; however, the present disclosure is not limited to these Examples, and many modifications can be made by those having ordinary knowledge in this field within the technical idea of the present disclosure.

Example 1: Evaluation of in vitro combined effect of compound 1 and selumetinib

A. Material and Method

Human lung cancer cell line A549 (obtained from Dainippon Pharmaceutical Co., Ltd.) was cultured in a DMEM medium containing 10% fetal bovine serum. The cells were maintained at 37°c and 5% $CO_2$, and passaged once or twice per week at a ratio of 1:5 to 1:20. A549 cells have been reported to have the G12S mutation in KRAS.

Cell Viability Assay

The cell viability was measured using crystal violet. After being collected according to a common method, the cells were suspended in the above medium containing 10% fetal bovine serum and seeded on a 12-well plate. The number of cells seeded was 1000 cells/800 μL per well. After the cells were incubated at 37°C and 5% $CO_2$ for 24 hours, 100 μL of a medium containing compound 1 and selumetinib or a vehicle was added.

In consideration of the sensitivity of the cells to each of the drugs, a 10-fold dilution series of two points, i.e., 10 and 100 nM, as well as a zero concentration (DMSO), was prepared for compound 1, and a 10-fold dilution series of three points, i.e., 0.1, 1, and 10 μM, as well as a zero concentration (DMSO), was prepared for selumetinib, to analyze the combinations.

After the addition of the drugs, the cells were further incubated at 37°C and 5% $CO_2$ for 10 days. During the incubation period, the medium was replaced and the drugs were added again three and seven days after the addition of the drugs. To determine the cell viability, a glutaraldehyde solution in an amount of 500 μL per well was added, and incubation was performed at room temperature for 20 minutes, followed by washing with tap water. After washing, a crystal violet solution in an amount of 500 μL per well was added, and incubation was performed at room temperature for 20 minutes, followed by washing with tap water. After washing, the plate was measured with a Gelcount plate reader. For quantification, dye extraction was performed using an extraction liquid (0.1N sodium dihydrogen phosphate/ethanol). Fig. 1 shows the results. The wavelength absorbance at 550 nm was measured with a SpectraMAX Plus 384 microplate reader, which is a plate reader. The cell viability when the drugs were added was calculated as a percentage according to the following formula, taking the control group as 100%.

Cell viability (%) = (absorbance when the drugs were added)/(absorbance of control group) × 100

The Fa (fraction of affect) value was calculated by subtracting 1/100 of the cell viability from 1.

Next, Bliss additivity values, which represent a calculated additive effect, were determined at each combined concentration according to the following formula, based on the effects of compound 1 and selumetinib as single drugs.

Bliss additivity value = (Fa value obtained from a single drug of compound 1) + (Fa value obtained from a single drug of selumetinib) - (Fa value obtained from a single drug of compound 1) × (Fa value obtained from a single drug of selumetinib)

The effect of combined use of the two drugs was determined according to the following formula; a judgement value of 10 or more indicates synergistic action, a value of -10 or more and less than 10 indicates additive action, and

a value of less than -10 indicates antagonistic action.

Judgment value = 100 × (Fa value obtained from actual combined effect) - (Bliss additivity value)

For the A549 cell line, the judgement values were calculated in terms of when compound 1 was used in combination with selumetinib. Table 1 shows the judgement values under each condition.

Table 1

| Compound 1 (nM) | | | | |
|---|---|---|---|---|
| 100 | | 48.2 | 18.8 | 1.2 |
| 10 | | 53.6 | 18.5 | 1.1 |
| 0 | | | | |
| | 0 | 0.1 | 1 | 10 |
| | | Selumetinib (nM) | | |

B: Results

The use of compound 1 in combination with selumetinib was confirmed to achieve an additional combined effect, showing positive values, in the region in which the concentration of compound 1 itself was 10 to 100 nM, according to the analysis using the Bliss additivity method.

Example 2: Evaluation of in vitro combined effect of compound 1 and trametinib, binimetinib, or cobimetinib

A. Material and Method

Human lung cancer cell line A549 (obtained from the American Type Culture Collection) and LU99 (obtained from the JCRB Cell Bank) were cultured in DMEM and RPMI media each containing 10% fetal bovine serum. The cells were maintained at 37°c and 5% $CO_2$, and passaged once or twice per week at a ratio of 1:5 to 1:20. A549 cells have been reported to have the G12S mutation in KRAS, and LU99 cells have been reported to have the G12C mutation in KRAS.

Cell Viability Assay

The cell viability was measured using crystal violet. After being collected according to a common method, the cells were suspended in the above media containing 10% fetal bovine serum, and seeded on a 24-well plate. The number of cells seeded per well was 1500 cells/500 μL (A549) and 3000 cells/500 μL (LU99). After the cells were incubated at 37°C and 5% $CO_2$ for 24 hours, the media were replaced with 500 μL of medium containing compound 1 and trametinib, binimetinib, cobimetinib, or a vehicle.

For compound 1, a dilution series of three points, i.e., 10, 30, and 100 nM, as well as a zero concentration (DMSO), was prepared. For trametinib, a dilution series of 5 points, i.e., 0.3, 1, 3, 10, and 30 μM, and for binimetinib and cobimetinib, a dilution series of 5 points, i.e., 3, 10, 30, 100, and 300 μM, as well as zero concentrations (DMSO), were prepared to analyze the combinations.

After the addition of the drugs, the cells were further incubated at 37°C and 5% $CO_2$ for 10 days. During the incubation period, the media were replaced with media containing the drugs three and seven days after the addition of the drugs. To determine the cell viability, a glutaraldehyde solution in an amount of 300 μL per well was added, and incubation was performed at room temperature for 20 minutes, followed by washing with tap water. After washing, a crystal violet solution in an amount of 500 μL per well was added, and incubation was performed at room temperature for 20 minutes, followed by washing with tap water. After washing, plate images were photographed. Fig. 2 shows the photographed images in terms of the A549 cell line, and Fig. 3 in terms of the LU99 cell line. For quantification, dye extraction was performed using an extraction liquid (0.1N sodium dihydrogen phosphate/ethanol), and the wavelength

absorbance at 490 nm was measured with a FlexStation 3 plate reader. The cell viability when the drugs were added was calculated as a percentage according to the following formula, taking the control group as 100%.

Cell viability (%) = (absorbance when the drugs were added)/(absorbance of control group) × 100

The Fa (fraction of affect) value was calculated by subtracting 1/100 of the cell viability from 1.

Next, Bliss additivity values, which represent a calculated additive effect, were determined at each combined concentration according to the following formula, based on the effects of compound 1, trametinib, binimetinib, and cobimetinib as single drugs.

Bliss additivity value = (Fa value obtained from a single drug of compound 1) + (Fa value obtained from a single drug of trametinib, binimetinib, or cobimetinib) - (Fa value obtained from a single drug of compound 1) × (Fa value obtained from a single drug of trametinib, binimetinib, or cobimetinib) The effect of combined use of the two drugs was determined according to the following formula; a judgement value of 10 or more indicates synergistic action, a value of -10 or more and less than 10 indicates additive action, and a value of less than -10 indicates antagonistic action.

Judgment value = 100 × (Fa value obtained from actual combined effect) - (Bliss additivity value)

For the A549 cell line, the judgement values were calculated in terms of when compound 1 was used in combination with trametinib, binimetinib, or cobimetinib; Table 2 shows the judgement values under each condition. For the LU99 cell line, the judgement values were calculated in terms of when compound 1 was used in combination with trametinib, binimetinib, or cobimetinib; Table 3 shows the judgement values under each condition.

Table 2

| Compound 1 (nM) | | | | | | |
|---|---|---|---|---|---|---|
| 100 | | -21. 3 | -6.4 | 2. 1 | 2 | 1 |
| 30 | | -20.8 | -2.5 | -0.6 | 5 | -3 |
| 10 | | -25.2 | -5.6 | -9.1 | 5.1 | -6.3 |
| 0 | | | | | | |
| | 0 | 0.3 | 1 | 3 | 10 | 30 |
| | | Trametinib (nM) | | | | |

| Compound 1 (nM) | | | | | | |
|---|---|---|---|---|---|---|
| 100 | | -18.5 | -19.7 | -1 | 2.8 | 0.8 |
| 30 | | -25.5 | -7 | -4.1 | 7 | -1.8 |
| 10 | | -20.3 | -10.7 | -3.2 | 7.8 | -1.3 |
| 0 | | | | | | |
| | 0 | 3 | 10 | 30 | 100 | 300 |
| | | Binimetinib (nM) | | | | |

| Compound 1 (nM) | | | | | | |
|---|---|---|---|---|---|---|
| 100 | | -19.9 | -20.1 | -4.6 | 6.3 | 6.4 |
| 30 | | -22.4 | -4.2 | -5.3 | 12.2 | 2.5 |
| 10 | | -18.4 | -8.9 | -5.6 | 11 | 4.3 |
| 0 | | | | | | |
| | 0 | 3 | 10 | 30 | 100 | 300 |
| | | Cobimetinib (nM) | | | | |

Table 3

| Compound 1 (nM) | | | | | |
|---|---|---|---|---|---|
| 100 | -7.3 | -7.3 | 4.9 | 9.8 | 2.7 |
| 30 | -14.5 | 1 | -2.3 | 12.7 | 1.1 |

(continued)

| Compound 1 (nM) | | | | | |
|---|---|---|---|---|---|
| 10 | -8.3 | -3.1 | -1.5 | 12 | -4.4 |
| 0 | | | | | |
| 0 | 0.3 | 1 | 3 | 10 | 30 |
| | Trametinib (nM) | | | | |

| Compound 1 (nM) | | | | | |
|---|---|---|---|---|---|
| 100 | -19.1 | -17.8 | -0.5 | 6.8 | 2.2 |
| 30 | -18.2 | 0.3 | -0.8 | 12.7 | 1.3 |
| 10 | -14.9 | -0.6 | 2, 7 | 9.1 | 2 |
| 0 | | | | | |
| 0 | 3 | 10 | 30 | 100 | 300 |
| | Binimetinib (nM) | | | | |

| Compound 1 (nM) | | | | | |
|---|---|---|---|---|---|
| 100 | -17.8 | -18 | -15.8 | 1.4 | 17 |
| 30 | -18.9 | -8.4 | -16.7 | 10.2 | 13.6 |
| 10 | -14.7 | -10 | -11.6 | 6.6 | 7.9 |
| 0 | | | | | |
| 0 | 3 | 10 | 30 | 100 | 300 |
| | Cobimetinib (nM) | | | | |

B: Results

In the A549 or LU99 cell line, the use of compound 1 in combination with trametinib resulted in judgement values of -10 or more in the region in which the concentration of compound 1 itself was 10 nM or more, and the concentration of trametinib was 10 nM or more, showing an additive effect in the A549 cell line and an additive or synergistic combined effect in the LU99 cell line, according to the analysis using the Bliss additivity method.

In the A549 or LU99 cell line, the use of compound 1 in combination with binimetinib resulted in judgement values of -10 or more in the region in which the concentration of compound 1 itself was 10 nM or more, and the concentration of binimetinib was 100 nM or more, showing an additive effect in the A549 cell line and an additive or synergistic combined effect in the LU99 cell line, according to the analysis using the Bliss additivity method.

In the A549 or LU99 cell line, the use of compound 1 in combination with cobimetinib resulted in judgement values of -10 or more in the region in which the concentration of compound 1 itself was 10 nM or more, and the concentration of cobimetinib was 100 nM or more, showing an additive or synergistic combined effect in both the A549 cell line and the LU99 cell line, according to the analysis using the Bliss additivity method.

The concentration of compound 1 of 10 nM corresponds to maintaining a blood concentration of 4.2 ng/mL in human pharmacokinetics for 24 hours in the case of administration every day and 48 hours in the case of administration intermittently.

A comparison of the above with the PK data of compound 1 at a QD dose (every day) of 4 mg, 8 mg, 16 mg, 20 mg, and 24 mg as disclosed in AACR2018, R. Bahleda et al., poster CT121 (Fig. 4) revealed that the dose of 8 mg or more maintains the blood concentration for about 16 hours or more and also achieves a higher 24-hour total AUC. This suggests that administration every day of compound 1 at a dose within the range of at least 8 mg to 24 mg would achieve additive or synergistic efficacy with the MEK inhibitors.

A comparison of the above was also made with the PK data of compound 1 at a QOD dose (once every two days) of 8 mg, 16 mg, 24 mg, 36 mg, 56 mg, 80 mg, 120 mg, 160 mg, and 200 mg (Fig. 4), which revealed that the dose of 24 mg or more maintains the blood concentration for about 36 hours or more and, at the same time, achieves a higher 48-hour total AUC. This suggests that administration intermittently of compound 1 at a dose within the range of at least 24 mg to 200 mg would achieve additive or synergistic efficacy with the MEK inhibitors.

The concentration of trametinib of 10 nM corresponds to maintaining a blood concentration of 1.3 ng/mL in human pharmacokinetics for 24 hours. A comparison of this with the PK data of trametinib at a dose of 0.5 mg, 1 mg, 1.5 mg, 2 mg, 2.5 mg, 3 mg, and 4 mg once daily as published in Lancet Oncol, 13, 773-81 (2012), revealed that administration of 0.5 mg or more per dose basically achieves a blood concentration of 1.3 ng/mL for 24 hours or more, as well as a higher 24-hour total AUC. This suggests that administration of trametinib at a dose within the range of at least 0.5 mg to 4 mg once daily would achieve additive or synergistic efficacy with compound 1.

The clinical dose of trametinib (Mekinist tablets) is 2 mg once daily, 1.5 mg once daily with a one-stage decrease, and 1 mg once daily with a two-stage decrease (interview form of Mekinist tablets). This suggests that administration of trametinib at a dose within the range of at least 0.5 to 2 mg once a day for consecutive days would achieve additive or synergistic efficacy with compound 1.

The concentration of binimetinib of 100 nM corresponds to maintaining a blood concentration of 18.45 ng/mL in human pharmacokinetics for at least 24 hours. A comparison of this with the PK data of binimetinib at a dose of 30 mg, 45 mg, and 60 mg twice daily as published in BJC, 116, 575-583 (2017), revealed that administration of 30 mg or more per dose basically achieves a blood concentration of 18.45 ng/mL for more than 8 hours, as well as a 8-hour total AUC not less than twice more than 18.45 ng/mL. This suggests that administration of binimetinib at a dose within the range of at least 30 mg to 60 mg twice daily would achieve additive or synergistic drug efficacy with compound 1.

The clinical dose of binimetinib (Mektovi tablets) is 45 mg twice daily, 30 mg twice daily with a one-stage decrease, and 15 mg twice daily with two-stage decrease (interview form of Mektovi tablets). This suggests that administration of binimetinib at a dose within the range of 30 to 45 mg twice daily would clinically achieve an additive or synergistic efficacy with compound 1.

The concentration of cobimetinib of 100 nM corresponds to maintaining a blood concentration of 53 ng/mL in human pharmacokinetics for at least 24 hours. This was compared with the PK data of cobimetinib at a dose of 10 mg, 20 mg, 40 mg, 60 mg, and 80 mg once daily as published in Inv New Drug, 34, 604-13 (2016), Supplementary Figure 2. It was revealed that in the steady-state PK, the dose of 40 mg in Stage 1 (21/7) basically achieved a concentration higher than the above for 14 hours, as well as a higher 24-hour total AUC. This suggests that administration of cobimetinib at a dose within the range of at least 40 mg to 60 mg once daily would achieve additive or synergistic efficacy with compound 1.

The clinical dose of cobimetinib (Cotellic tablets) is 60 mg, once daily for 21 days per 28-day cycle, 40 mg once daily for 21 days per 28-day cycle with a one-stage decrease, and 20 mg once daily for 21 days per 28-day cycle with a two-stage decrease (data sheet of Cotellic tablets). This suggests that administration of cobimetinib at a dose within the range of 40 to 60 mg once daily would clinically achieve additive or synergistic efficacy with compound 1.

## Claims

1. An antitumor agent comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as an active ingredient, the antitumor agent being administered in combination with an MEK inhibitor.

2. The antitumor agent according to claim 1, wherein the MEK inhibitor is trametinib or a salt thereof.

3. The antitumor agent according to claim 1, wherein the MEK inhibitor is binimetinib or a salt thereof.

4. The antitumor agent according to claim 1, wherein the MEK inhibitor is cobimetinib or a salt thereof.

5. The antitumor agent according to claim 2, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered at a dose selected from the group consisting of 8 mg, 12 mg, 16 mg, and 20 mg per dose once daily, and trametinib or a salt thereof is administered at a dose selected from the group consisting of 1 mg, 1.5 mg, and 2 mg per dose once daily.

6. The antitumor agent according to claim 3, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered at a dose selected from the group consisting of 8 mg, 12 mg, 16 mg, and 20 mg per dose once daily, and binimetinib or a salt thereof is administered at a dose selected from the group consisting of 30 mg and 45 mg per dose twice daily.

7. The antitumor agent according to claim 4, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered at a dose selected from the group consisting of 8 mg, 12 mg, 16 mg, and 20 mg per dose once daily, and cobimetinib or a salt thereof is administered at a dose selected from the group consisting of 40 mg and 60 mg per dose once daily.

8. The antitumor agent according to claim 2, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is intermittently administered at a dose selected from the group consisting of 24 mg, 36 mg, 56 mg, 80 mg, 120 mg, and 160 mg per dose, and trametinib or a salt thereof is administered at a dose selected from the group consisting of 1 mg, 1.5 mg, and 2 mg per dose once

daily.

9. The antitumor agent according to claim 3, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is intermittently administered at a dose selected from the group consisting of 24 mg, 36 mg, 56 mg, 80 mg, 120 mg, and 160 mg per dose, and binimetinib or a salt thereof is administered at a dose selected from the group consisting of 30 mg and 45 mg per dose twice daily.

10. The antitumor agent according to claim 4, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is intermittently administered at a dose selected from the group consisting of 24 mg, 36 mg, 56 mg, 80 mg, 120 mg, and 160 mg per dose, and cobimetinib or a salt thereof is administered at a dose selected from the group consisting of 40 mg and 60 mg per dose once daily.

11. The antitumor agent according to any one of claims 1 to 10, wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered before, simultaneously with, or after administration of the MEK inhibitor.

12. The antitumor agent according to any one of claims 1 to 11, wherein the tumor has an aberration in an RAS/MAPK signaling pathway.

13. The antitumor agent according to any one of claims 1 to 12, wherein the tumor is at least one member selected from the group consisting of head and neck cancer, gastroenterological cancer, lung cancer, breast cancer, genital cancer, a hematopoietic organ tumor, a bone and soft tissue tumor, skin cancer, and a brain tumor.

14. The antitumor agent according to any one of claims 1 to 13, wherein the tumor is lung cancer, pancreatic cancer, colorectal cancer, or a brain tumor.

15. An antitumor effect enhancer for enhancing an antitumor effect of an MEK inhibitor, the antitumor effect enhancer comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as an active ingredient.

16. An antitumor agent for use in the treatment of a cancer patient to whom an MEK inhibitor has been administered, the antitumor agent comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof as an active ingredient.

17. A pharmaceutical composition for use in the treatment of a tumor by administering the pharmaceutical composition in combination with an MEK inhibitor, the pharmaceutical composition comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and a pharmaceutical carrier as active ingredients.

18. (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof, for use in combination therapy with an MEK inhibitor in the treatment of a tumor.

19. A combination of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and an MEK inhibitor for use in the treatment of a tumor.

20. A method for treating a tumor, comprising administering a therapeutically effective amount of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and a therapeutically effective amount of an MEK inhibitor to a patient in need thereof.

21. A method for enhancing an antitumor effect of an MEK inhibitor, comprising administering a therapeutically effective amount of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof to a patient to whom the MEK inhibitor has been administered.

22. A method for treating a tumor, comprising administering a therapeutically effective amount of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof to a patient to whom an MEK inhibitor has been administered.

23. Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in the treatment of a tumor in a combination therapy with an MEK inhibitor.

**24.** Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in the enhancement of an antitumor effect of an MEK inhibitor.

**25.** Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in the treatment of a cancer patient to whom an MEK inhibitor has been administered.

**26.** Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in the production of an antitumor agent for use in a combination therapy with an MEK inhibitor.

**27.** Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in the production of an antitumor effect enhancer for an MEK inhibitor.

**28.** Use of a combination of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and an MEK inhibitor in the treatment of a tumor.

**29.** Use of a combination of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof and an MEK inhibitor in the production of an antitumor agent.

Fig. 1

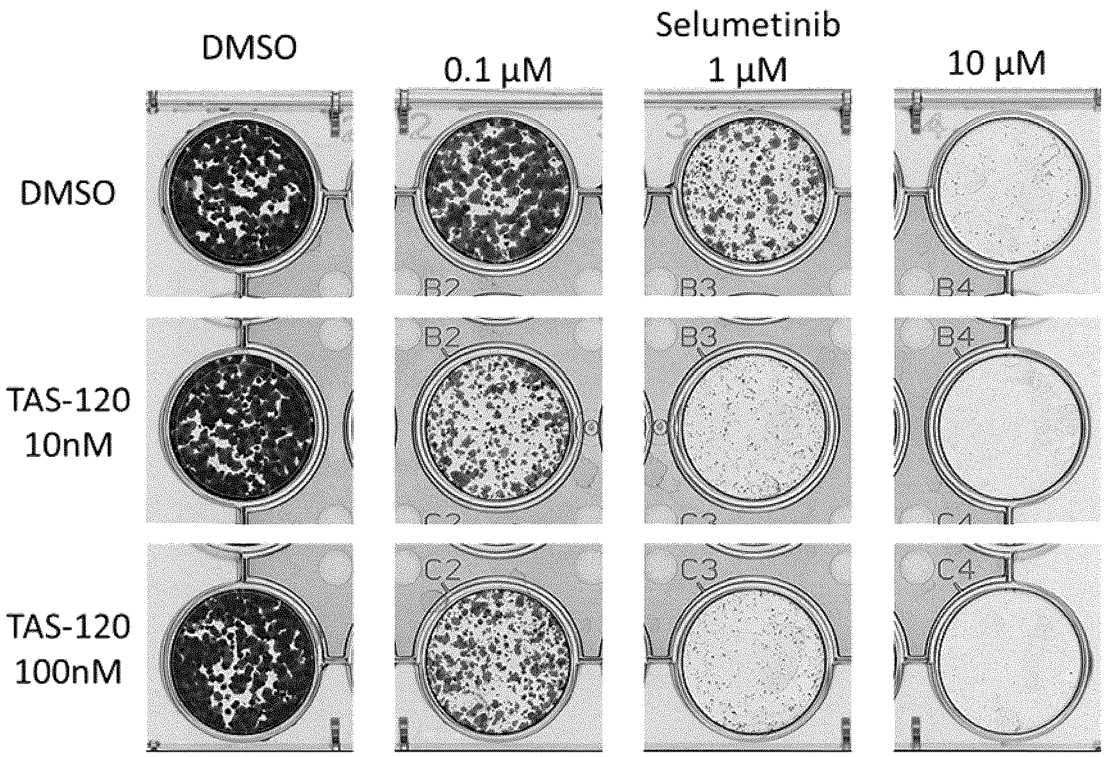
DMSO
Selumetinib
0.1 μM
1 μM
10 μM
DMSO
TAS-120
10nM
TAS-120
100nM

Fig. 2

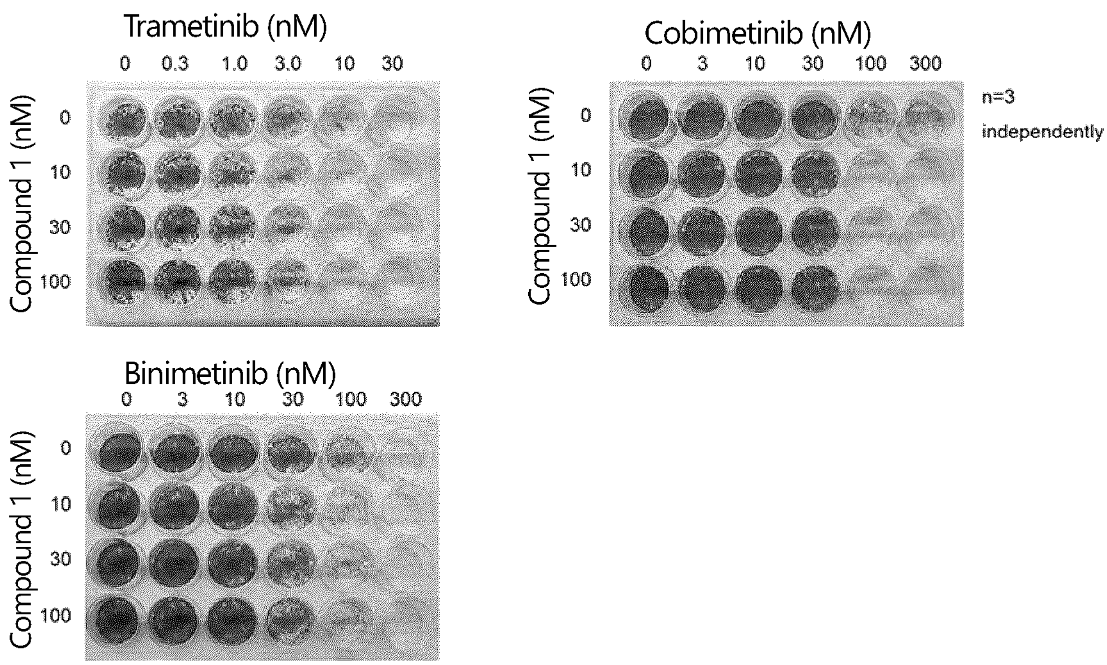
Trametinib (nM)
0 0.3 1.0 3.0 10 30
Compound 1 (nM)
0
10
30
100
Cobimetinib (nM)
0 3 10 30 100 300
Compound 1 (nM)
0
10
30
100
n=3
independently
Binimetinib (nM)
0 3 10 30 100 300
Compound 1 (nM)
0
10
30
100

Fig. 3

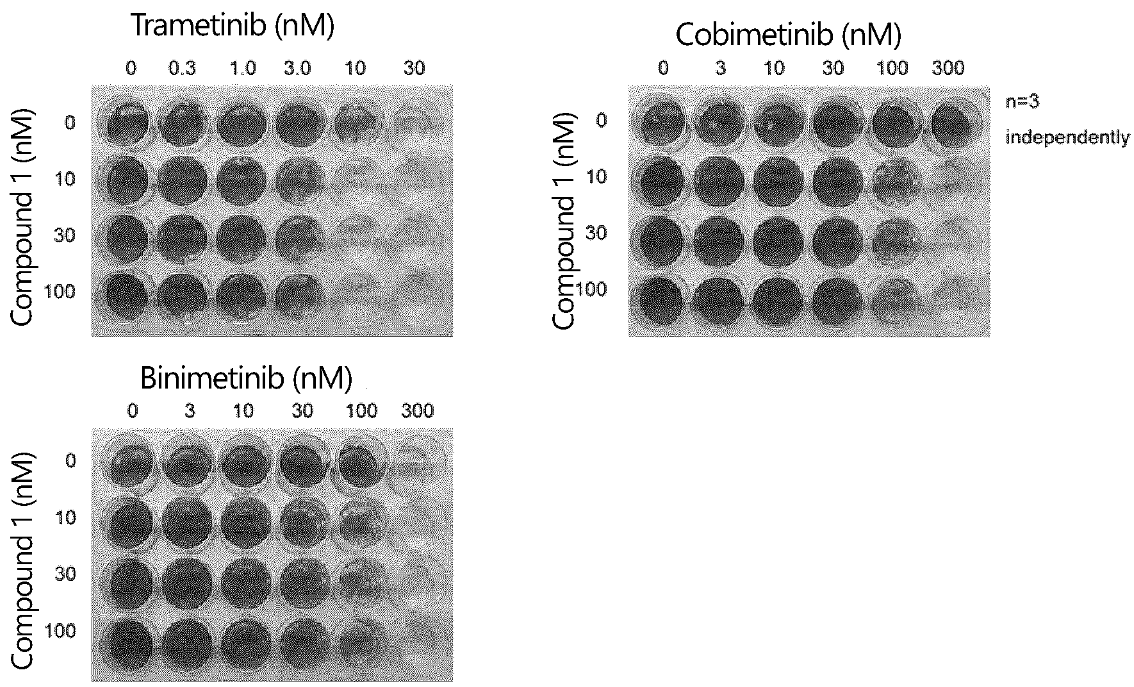
Trametinib (nM)
0 0.3 1.0 3.0 10 30
Compound 1 (nM)
0 10 30 100
Cobimetinib (nM)
0 3 10 30 100 300
Compound 1 (nM)
0 10 30 100
n=3
independently
Binimetinib (nM)
0 3 10 30 100 300
Compound 1 (nM)
0 10 30 100

Fig. 4

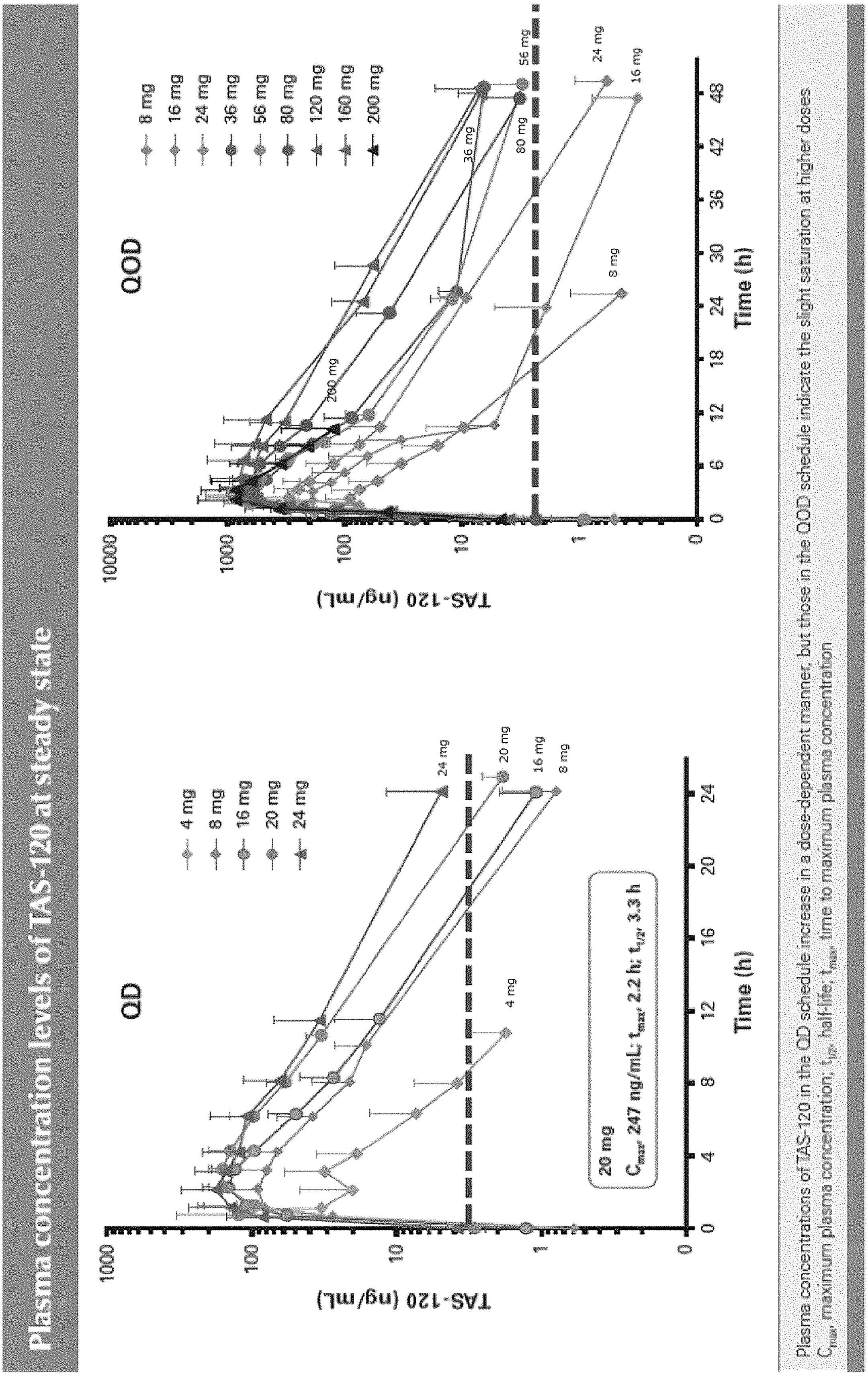

Pharmacokinetics/pharmacodynamics
Plasma concentration levels of TAS-120 at steady state
QD
TAS-120 (ng/mL)
Time (h)
4 mg
8 mg
16 mg
20 mg
24 mg
20 mg
Cmax, 247 ng/mL; tmax, 2.2 h; t1/2, 3.3 h
QOD
8 mg
16 mg
24 mg
36 mg
56 mg
80 mg
120 mg
160 mg
200 mg
Plasma concentrations of TAS-120 in the QD schedule increase in a dose-dependent manner, but those in the QOD schedule indicate the slight saturation at higher doses
Cmax, maximum plasma concentration; t1/2, half-life; tmax, time to maximum plasma concentration

**INTERNATIONAL SEARCH REPORT**

International application No. PCT/JP2021/015032

A. CLASSIFICATION OF SUBJECT MATTER
A61K 31/519(2006.01)i; A61K 31/4184(2006.01)i; A61K 31/4523(2006.01)i; A61K 45/00(2006.01)i; A61P 35/00(2006.01)i; A61P 43/00(2006.01)i; C07D 487/04(2006.01)i
FI: A61K31/519; A61K31/4184; A61K31/4523; A61P43/00 121; A61P35/00; A61K45/00; C07D487/04 143
According to International Patent Classification (IPC) or to both national classification and IPC

B. FIELDS SEARCHED
Minimum documentation searched (classification system followed by classification symbols)
A61K31/519; A61K31/4184; A61K31/4523; A61K45/00; A61P35/00; A61P43/00; C07D487/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| Y | KALYUKINA, M. et al., "TAS-120 Cancer Target Binding:Defining Reactivity and I Revealing the First Fibroblast Growth Factor Receptor 1 (FGFR1) Irreversible Structure", ChemMedChem 14 (2019), 494-500 Abstract, Introduction, fig. 1 | 1-29 |
| Y | MERIC-BERNSTAM, Fetal., "Efficacy of TAS-120, an irreversible fibroblast growth factor receptor (FGFR) inhibitor, in cholangiocarcinoma patients with FGFR pathway alternations who were previously treated with chemotherapy and other FGFR inhibitors", Annals of Oncology 29 (Supplement 5) (2018), v100—v110 whole document | 1-29 |
| Y | WO 2015/008839 A1 (TAIHO PHARMACEUTICAL CO., LTD.) 22 January 2015 (2015-01-22) claims, test examples | 1-29 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 April 2021 (22.04.2021) | 11 May 2021 (11.05.2021) |
| Name and mailing address of the ISA/ Japan Patent Office 3-4-3, Kasumigaseki, Chiyoda-ku, Tokyo 100-8915, Japan | Authorized officer<br>Telephone No. |

Form PCT/ISA/210 (second sheet) (January 2015)

INTERNATIONAL SEARCH REPORT

International application No. PCT/JP2021/015032

C (Continuation). DOCUMENTS CONSIDERED TO BE RELEVANT

| Category* | Citation of document, with indication, where appropriate, of the relevant passages | Relevant to claim No. |
|---|---|---|
| Y | BOCKORNY, B. et al., "RAS-MAPK Reactivation Facilitates Acquired Resistance 1-29 in FGFR1-Amplified Lung Cancer and Underlies a Rationale for Upfront FGFR-MEK Blockade", Molecular Cancer Therapeutics 17(7) (2018), 1526-1539 Abstract, Introduction, fig. 5-6, Discussion | 1-29 |

Form PCT/ISA/210 (continuation of second sheet) (January 2015)

**INTERNATIONAL SEARCH REPORT**
Information on patent family members

International application No.
PCT/JP2021/015032

| Patent Documents referred in the Report | Publication Date | Patent Family | Publication Date |
|---|---|---|---|
| WO 2015/008839 A1 | 22 Jan. 2015 | US 2016/0193210 A1<br>claims, examples<br>EP 3023100 A1<br>KR 10-2016-0032186 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2020071355 A
- WO 2013108809 A
- WO 2017150725 A
- WO 2016130917 A
- WO 2017086332 A
- WO 2015008839 A
- WO 2005121142 A
- WO 2005051906 A
- WO 20007044515 A
- WO 2003077914 A
- WO 2006045514 A
- WO 2002006213 A
- WO 2007014011 A

### Non-patent literature cited in the description

- *Nat. Rev. Cancer,* 2010, vol. 10, 116-129
- *J. Clin. Oncol.,* 2006, vol. 24, 3664-3671
- *Mol. Cancer Res.,* 2005, vol. 3, 655-667
- *Cancer Res.,* 2010, vol. 70, 2085-2094
- *Cellular Physiology and Biochemistry,* 2014, vol. 33, 633-645
- *Lancet Oncol,* 2012, vol. 13, 773-81
- *BJC,* 2017, vol. 116, 575-583
- *Inv New Drug,* 2016, vol. 34, 604-13